Europäisches Patentamt
European Patent Office
Office européen des brevets

(19)

(11) Publication number: **0 075 805**
**A1**

(12) # EUROPEAN PATENT APPLICATION

(21) Application number: 82108606.3

(22) Date of filing: 17.09.82

(51) Int. Cl.$^3$: **C 07 D 501/20**
C 07 D 471/04, C 07 D 501/34
C 07 D 501/36, C 07 D 501/56
C 07 D 277/40, C 07 D 277/42
C 07 D 277/44, A 61 K 31/425
A 61 K 31/435, A 61 K 31/545
//(C07D471/04, 221/00, 205/00)

(30) Priority: 18.09.81 JP 147250/81

(43) Date of publication of application: 06.04.83 Bulletin 83/14

(84) Designated Contracting States: DE FR GB IT

(71) Applicant: KYOWA HAKKO KOGYO CO., LTD
Ohtemachi Bldg. Ohtemachi 1-chome Chiyoda-ku
Tokyo(JP)

(72) Inventor: Hirata, Tadashi
1566-315, Hara-cho Midori-ku
Yokohama-shi Kanagawa-ken(JP)

(72) Inventor: Ogasa, Takehiro
3-6-6, Asahi-machi
Machida-shi Tokyo(JP)

(72) Inventor: Mochida, Kenichi
325-5, Sanada
Hiratsuka-shi Kanagawa-ken(JP)

(72) Inventor: Suzuki, Fumio
1566-47, Nara-cho Midori-ku
Yokohama-shi Kanagawa-ken(JP)

(72) Inventor: Yamazaki, Motoo
3-6-6, Asahi-machi
Machida-shi Tokyo(JP)

(72) Inventor: Sato, Kiyoshi
58-1, Kamo Mishima-shi
Shizuoka-ken(JP)

(74) Representative: Casalonga, Axel et al,
BUREAU D.A. CASALONGA OFFICE JOSSE & PETIT
Baaderstrasse 12-14
D-8000 München 5(DE)

(54) **Beta-lactam compound and a pharmaceutical composition containing the same.**

(57) β-Lactam compounds represented by the general formula:

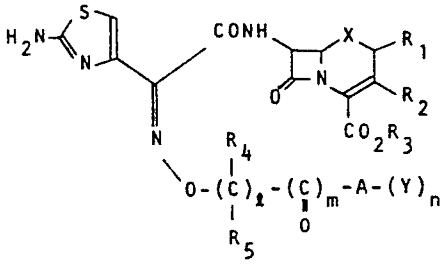

(wherein X represents $CH_2$, S or O; Y represents a hydroxyl group, lower alkanoyloxy group, amino group or substituted amino group; A represents a phenyl group, naphthyl group, or monocyclic or dicyclic heterocyclic group, respectively substituted by $(Y)_n$; $R_4$ and $R_5$ each represent a hydrogen atom, lower alkyl group or cycloalkylidene group together with the carbon atom combined therewith; $\ell$ represents an integer of 0, 1, 2 or 3; m represents an integer of 0 or 1; and n represents an integer of 1, 2, 3, 4 or 5; and $R_1$, $R_2$ and $R_3$ are groups often used in the conventional β-lactam compounds) have strong antimicrobial activities against a wide range of Gram positive and negative bacteria; particularly Gram negative bacteria including *Pseudomonas aeruginosa*, *Pseudomonas sepatia* and various *Enterobacters*.

EP 0 075 805 A1

## TITLE OF INVENTION

β-LACTAM COMPOUND AND A PHARMACEUTICAL COMPOSITION CONTAINING THE SAME

### Background of the Invention

The present invention relates to a β-lactam compound wherein a specific acyl group, aralkyl group, etc., are substituted for hydrogen atom of the hydroxyimino group of a cephalosporin or an analog thereof (containing carbacephem skeleton) having a 7-[2-(2-aminothiazol-4-yl)-2-syn-hydroxyimino] acetamido group, a related compound thereof, and a pharmaceutical composition containing said β-lactam compound.

Many β-lactam antibiotics have been and are under development in order to provide good chemical medicaments for curing various microbial infections. Among those compounds, β-lactam compounds wherein various groups are substituted for hydrogen atom of the hydroxyimino group of a cephalosporin or an analog thereof having a 7-[2-(2-aminothiazol-4-yl)-2-syn-hydroxyimino] acetamido group, have been known (US Patent Nos. 4,209,616, 4,165,430, 4,202,893, GB 2017103A, EP 0014476 Al, etc.).

It has initially been found that the β-lactam compounds of the present invention have strong antimicrobial activities against a wide range of Gram positive and negative bacteria, particularly Gram negative bacteria including Pseudomonas aeruginosa, Pseudomonas sepatia and various Enterobacters.

### Detailed Description of the Invention

This invention relates to a novel β-lactam compound represented by the general formula (I):

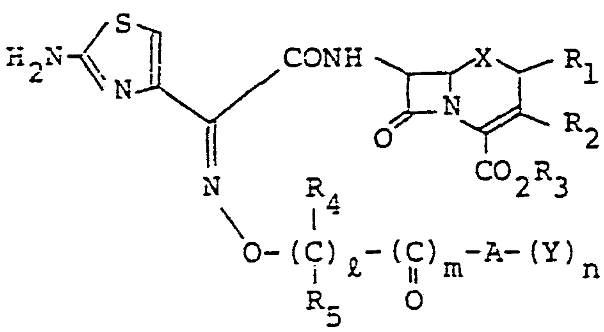

( I )

{wherein X represents $CH_2$ (methylene group), S (sulfur atom), or O (oxygen atom); Y represents a hydroxyl group or lower alkanoyloxy group; A represents a phenyl group or naphthyl group, respectively substituted by $(Y)_n$; $R_1$ represents a hydrogen atom, hydroxyl group, methoxy group or lower alkyl group; $R_2$ represents a hydrogen atom, halogen atom, methoxy group or $CH_2R_2'$ [wherein $R_2'$ represents a hydrogen atom, azido group, lower alkanoyloxy group, carbamoyloxy group, pyridinium group, substituted pyridinium group, or substituted or unsubstituted heterocyclic thio group (wherein the term "heterocyclic" means a 5-membered or 6-membered heterocyclic group having 1 to 4 hetero atoms of O, S and N)]; $R_3$ represents a hydrogen atom, alkali metal, alkaline earth metal, organic ammonium group or ester residue; $R_4$ and $R_5$ each represent a hydrogen atom, lower alkyl group or cycloalkylidene group together with the carbon atom combined therewith; ℓ represents an integer of 0, 1, 2 or 3; m represents an integer of 0 or 1; and n represents an integer of 1, 2, 3, 4 or 5}. The present invention further relates to a novel carboxylic acid represented by the general formula (II):

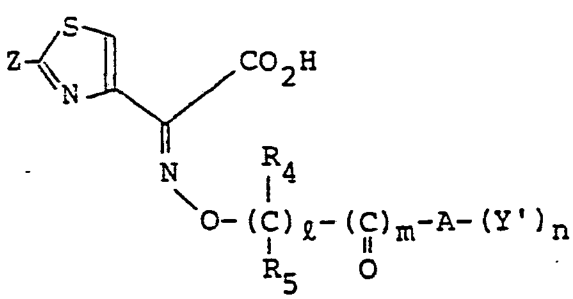

( II )

(wherein Z represents an amino group or protected amino group, Y' represents the same Y as defined above, or a protected hydroxyl group, and A, $R_4$, $R_5$, ℓ, m and n have the same meanings as defined above), and its reactive derivative.

In "Y" of the compound represented by the general formula (I), the lower alkanoyloxy group means a straight or branched lower alkanoyloxy group having 1 to 6 carbon atom.

In the $R_1$ group, the lower alkyl group means a straight or branched lower alkyl group having 1 to 6 carbon atoms. In the $R_2$ group, the halogen atom includes chlorine, bromine, etc. In the $R_2'$ group, the lower alkanoyloxy group means a lower alkanoyloxy group having 1 to 6 carbon atoms, and the substituent for the substituted pyridinium group includes $-(CH_2)_p-R_A$ (wherein $R_A$ is a carbamoyl group, carboxyl group, cyano group or lower alkoxycarbonyl group having 2 to 5 carbon atoms, and p is 0, 1, 2 or 3), $-R_B-SO_3-R_C$ (wherein $R_B$ is a lower alkylene group having 1 to 3 carbon atoms, and $R_C$ has the same meaning as $R_3$), etc. The substituent for the heterocyclic thio group includes, for example, a lower alkyl group having 1 to 5 carbon atoms, hydroxyl group, oxo group, amino group, nitro group, $-(CH_2)_qCO_2H$, $-(CH_2)_qCO_2$—(lower alkyl group), $-(CH_2)_qSO_3H$, $-(CH_2)_q-N-$(lower alkyl group)$_2$, etc., wherein q is an integer of 1 to 3 and the lower alkyl group means a lower alkyl group having 1 to 4 carbon atoms. The 5-membered or 6-membered heterocyclic group having 1 to 4 hetero atoms of O, S and N is preferably exemplified by a tetrazolyl group, thiadiazolyl group, triazolyl group, triazinyl group, thiazolyl group, etc.

In the $R_3$ group, the alkali metal and the alkaline earth metal mean a metal capable of forming a counter cation of carboxylate anion ($COO^-$), and the former includes, for example, sodium, potassium, etc., and the latter includes, for example, magnesium, calcium, barium, etc. The counter cation group also includes, for example, an ammonium group of organic amines such as basic amino acids. The ester residue represented by "$R_3$" includes, for example, a group which can be comparatively readily eliminable in a living body, represented by the formula:

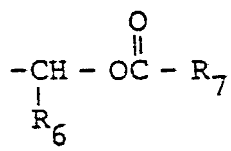

(wherein $R_6$ represents a hydrogen atom or lower alkyl group having 1 to 6 carbon atoms, and $R_7$ represents a lower alkyl group having 1 to 6 carbon atoms or phenyl group), or by the formula:

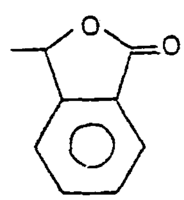

In $R_4$ and $R_5$, the lower alkyl group means a lower alkyl group having 1 to 5 carbon atoms, and the cycloalkylidene group means a cycloalkylidene group having 3 to 6 carbon atoms.

In the general formula (II), Z represents an amino group or a protected amino group, and the protective group is exemplified by the ordinary protective groups for amino group, such as a trityl group, formyl group, chloroacetyl group, bromoacetyl group, 2,2,2-trichloroacetyl group, t-butyloxycarbonyl group, benzyloxycarbonyl group, etc.

In Y', the protective group of the protected hydroxyl group is exemplified by a formyl group, chloroacetyl group, bromoacetyl group, 2,2,2-trichloroethoxycarbonyl group, tetrahydropyranyl group, methoxymethyl group, ethoxyethyl group, trimethylsilyl group, t-butyl-dimethylsilyl group, etc. When at least two adjacent hydroxyl groups such as in catechol, etc. come into question, the protective group also includes ones capable of forming such cyclic protected compounds, as given by the following formulae:

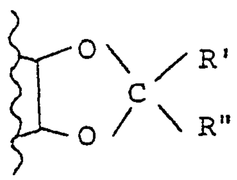 , 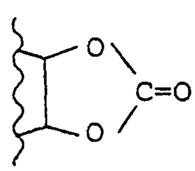 or 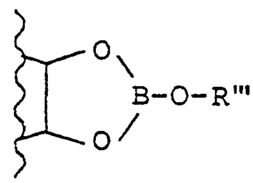

(wherein R' and R" are the same or different and represent hydrogen atoms, lower alkyl groups having 1 to 6 carbon atoms or phenyl groups, and R"' represents a hydrogen atom or lower alkyl group having 1 to 3 carbon atoms). The protective group of the protected amino group is exemplified by a formyl group, acetyl group, chloroacetyl group, bromoacetyl group, 2,2,2-trichloroethoxycarbonyl group, t-butyloxycarbonyl group, etc.

The reactive derivative of carboxylic acid represented by the general formula (II) is exemplified by an acid halide such as acid chloride and acid bromide, an acid anhydride, mixed acid anhydride such as one with a chlorocarbonic ester, e.g. methyl chlorocarbonate, ethyl chlorocarbonate, isobutyl chlorocarbonate, etc., acid azide, active ester such as nitrophenylthio ester and N-hydroxysuccinic acid ester, etc., and means all the carboxylic acid derivatives capable of forming an acyl compound with the amino group at the 7-position of cephalosporin or its analogs.

The β-lactam compound represented by the general formula (I) can be prepared according to the following procedures:

Procedure (A)

carboxylic acid represented by the general formula (II) or its reactive derivative +

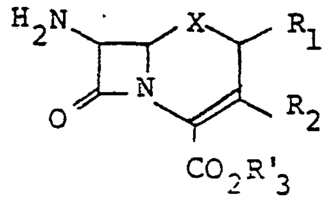

(III)

condensation

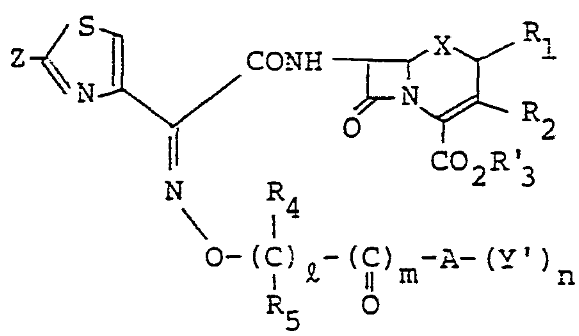

(IV)

removal of protective group

compound represented by the general formula (I)

(wherein $R'_3$ represents the same $R_3$ as defined above, a t-butyl group, benzyl group, p-nitrobenzyl group, benzhydryl group or trityl group).

The ordinary method for acylation of penicillin and cephalosporin is applicable to the condensation in the first step, and includes an acid halide method, an acid anhydride method (including a dicyclohexylcarbodiimide method), mixed acid anhydride method, active ester method and acid azide method as the main ones. In that case, an acid chloride of carboxylic acid (II) can be prepared by reacting carboxylic acid (II) with a chlorinating agent such as phosphorus pentachloride, thionyl chloride and oxalyl chloride. The 7-amino compound (III) can be used as such or as an inorganic acid salt such as hydrochloride, hydrobromide and phosphate, or as an organic acid salt such

as trifluoroacetate and methanesulfonate, or as an N-silyl derivative.

The acylation (condensation) reaction is carried out in an aqueous or non-aqueous solvent at a temperature of -50 to +50°C, if necessary, in the presence of a base or other acid-capturing agent. Halogenated hydrocarbon such as methylene chloride, ether such as tetrahydrofuran, ester such as ethyl acetate, amide such as dimethylformamide, sulfoxide such as dimethylsulfoxide, acetonitrile, acetone, water, etc. can be used alone or in combination thereof.

Organic tertiary amine such as triethylamine and dimethylaniline, inorganic base such as sodium bicarbonate and potassium carbonate can be used as the base. Oxyrane compound such as ethylene oxide and propylene oxide can be used as the acid-capturing agent.

Among the compounds represented by the general formula (III), cephalosporins wherein X is S are well known compounds, and oxacephalosporins wherein X is O can be prepared according to the procedures disclosed in J. Med. Chem., 20, 551 (1977), ibid. 22 757 (1979), J. Am. Chem. Soc., 101, 4403 (1979), etc. Carbacephems wherein X is $CH_2$ can be prepared according to the procedures disclosed in GB 2017102A, GB 2017103A, US Patent SN 107,435 filed on December 26, 1979, EP 0014475A1, EP 0027882A1, etc.

The second step is a step for removing the protective group, where, after the isolation and purification of the intermediate (IV), or without the isolation and purification, the protective group for the functional group (hydroxyl group, amino group and carboxyl group) in Z, Y' and $R'_3$ are removed according to the conventional procedure. Suitable procedures are applied for removing the respective protective group for the hydroxyl group, amino group and carboxyl group, and removal of the individual protective groups can be carried out according to the procedures described in detail in chapters 2, 3, 4 and 5 of "Protective Groups in Organic Chemistry, compiled by Mc Omie (1973, Plenum Press).

Procedure (B)

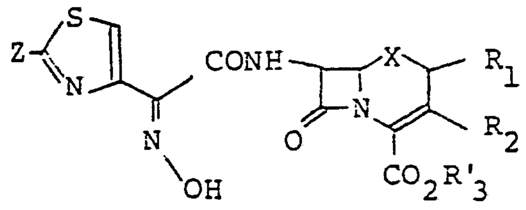
+
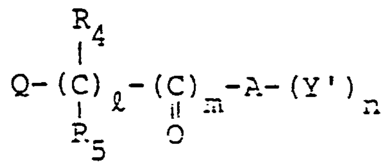

(V) (VI)

condensation

compound represented by the general formula (IV)

Removal of protective group

compound represented by the general formula (I)

The first step is a step of condensing a hydroxyimino compound represented by the general formula (V) with a reactive alkylating agent represented by the general formula (VI) ($\ell \neq 0$) or an acylating agent ($\ell = 0$, $m = 1$).

In the case of $\ell \neq 0$, Q represents a chlorine atom, bromine atom or iodine atom, or a sulfonate group such as mesyloxy group and tosyloxy group. In the case of $\ell = 0$ and $m = 1$, compound (VI) represents a reactive derivative of corresponding carboxylic acid as $Q-\underset{O}{\underset{\|}{C}}-A-(Y')_n$. The reactive derivative is exemplified by an acid halide, acid anhydride (including the ones formed by carbodiimide, etc.) mixed acid anhydride, active ester, etc. As the acid halide, mixed acid anhydride and active ester, similar ones as described in respect of the compound of the formula (II) are exemplified.

Condensation can be carried out in a solvent in a temperature range of -50 to +50°C, if necessary, in the presence of a base. To suppress formation of anti-isomer, it is preferable to carry out condensation below 5°C.

The same bases and solvents as used in procedure (A) can be used. The compounds represented by the general formula (V) are well known, and a process for preparing the

compound of $X = CH_2$ is disclosed in Japanese Published Unexamined Patent Application No. 49381/81. A group of compounds represented by the general formula (VI) are also well known [see Chemische Berichte 61B 791 (1928)].

The carboxylic acid represented by the general formula (II) can be prepared according to the following procedure:

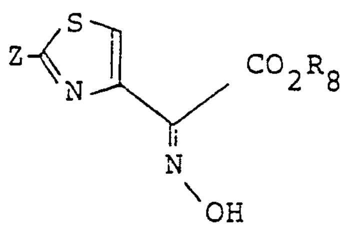

(VII)

+

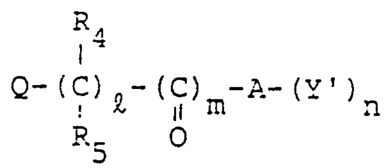

(VI)

Condensation and, if necessary, removal of carboxyl-protecting group

Compound represented by the general formula (II)

(where $R_8$ represents a hydrogen atom or a carboxyl-protecting group)

The condensation reaction can be carried out in the same manner as in the procedure B for preparing the compound represented by the general formula (I). In the case of $\ell \neq 0$, Q is a chlorine atom, bromine atom or iodine atom, or a sulfonate group such as mesyloxy group and tosyloxy group. In the case of $\ell = 0$ and $m = 1$, compound (VI) is a reactive derivative of corresponding carboxylic acid as $Q-\underset{\underset{O}{\|}}{C}-A-(Y')_n$.

The reactive derivative is exemplified by an acid halide, acid anhydride (including the ones formed by carbodiimide, etc.), mixed acid anhydride, active ester, etc. Condensation can be carried out in the same solvent as used in the said procedure (B) in a temperature range of -50 to +50°C, if necessary, in the presence of a base. To suppress formation of an anti-isomer, it is desirable to carry out

condensation below 5°C.

The carboxyl-protecting group $R_8$ is exemplified, in case of $\ell \neq 0$ (except, $\ell \neq 1$ in case of $m = 1$ and $R_4 = R_5 = 0$), by a group removable by an alkali, acid or a Lewis acid, for example, a lower alkyl group such as methyl, ethyl and t-butyl, an arylmethyl group such as benzyl, p-methoxybenzyl, p-nitrobenzyl, diphenylmethyl and triphenylmethyl, etc., and in the case of $\ell = 0$ and $m = 1$ or in case of $\ell = 1$ and $m = 1$ and $R_4 = R_5 = H$, by t-butyl, benzyl, p-methoxybenzyl, p-nitrobenzyl, diphenylmethyl, triphenylmethyl, etc., which can be removed by an acid or Lewis acid.

Reaction of removing carboxyl-protecting group can be carried out in a temperature range of -50 to +50°C under the ordinary deesterification conditions. To avoid isomerization into anti-isomer, it is desirable to carry out the reaction below 10°C. Of course, the condensation reaction can be carried out even by free carboxylic acid wherein $R_8$ is a hydrogen atom.

The present β-lactam compounds represented by the general formula (I) have strong antimicrobial activities against Gram positive and Gram negative bacteria and are useful for curing various infections, as a bactericide or as a component of a disinfectant.

Thus, the invention includes within its scope pharmaceutical compositions comprising, as an active ingredient, a β-lactam compound represented by the general formula (I) or a pharmaceutically acceptable salt thereof in association with a pharmaceutical carrier or diluent. The compounds of this invention are administered by parenteral (intramuscular, intraperitoneal, intravenous or subcutaneous injection routes), oral or rectal route of administration and can be formulated in dosage forms appropriate for each route of administration.

Preparations according to this invention for parenteral administration include sterile aqueous or non-aqueous solutions, suspensions or emulsions. Examples of non-aqueous solvents or vehicles are propylene glycol,

polyethylene glycol, vegetable oils such as olive oil, and injectable organic esters such as ethyl oleate. Such dosage forms may also contain adjuvants such as preserving, wetting, emulsifying and dispersing agents. They may be sterilized by, for example, filtration through a bacteria-retaining filter, by incorporating sterilizing agents into the compositions, by irradiating the compositions or by heating the compositions. They can also be manufactured in the form of sterile solid compositions which can be dissolved in sterile water or some other sterile injectable medium immediately before use.

Compositions for oral administration may be presented in a form suitable for absorption by the gastrointestinal tract. Tablets and capsules for oral administration may be in unit dose presentation form, and may contain conventional excipients such as binding agents, for example, syrup, acacia, gelatin, sorbitol, tragacanth or polyvinylpyrrolidone; fillers, for example, lactose, sugar, maize-starch, calcium phosphate, sorbitol or glycine; lubricants, for example, magnesium stearate, talc, polyethylene glycol, silica; disintegrants, for example, potato starch or acceptable wetting agent such as sodium lauryl sulphate. The tablets may be coated according to methods well known in the arts. Oral liquid preparations may be in the form of aqueous or oily suspension, solution, emulsions, syrups, etc., or may be presented as a dry product, for reconstitution with water or other suitable vehicle before use. Such liquid preparations may contain conventional additive such as suspending agents, for example, sorbitol syrup, methyl cellulose, glucose sugar syrup, gelatin, hydroxyethylcellulose, carboxymethylcellulose, aluminum stearate gel, emulsifying agents, for example, lecithin or sorbitan monooleate; non-aqueous vehicles, which may include edible oils, for example, almond oil, coconut oil, propylene glycol or ethyl alcohol; preservatives, for example, methyl or propyl p-hydroxybenzoates or sorbic acid.

Compositions for rectal administration are preferably suppositories which may contain, in addition to the

active substance, excipients such as cocoa butter or a suppository wax.

The dosage of active ingredient in the compositions of this invention may be varied; however, it is necessary that the amount of the active ingredient shall be such that a suitable dosage form is obtained. The selected dosage depends upon the desired therapeutic effect, on the route of administration, and on the duration of the treatment. Generally, dosage levels of between 5 and 350 mg/kg of body weight daily are administered to mammalian patients to achieve an antibiotic effect.

The present invention is described in detail below, referring to Examples and Reference Examples.

Example 1

Preparation of 2-(2-tritylaminothiazole-4-yl)-2-syn-(3,4-diacetoxybenzoyloxyimino) acetic acid:

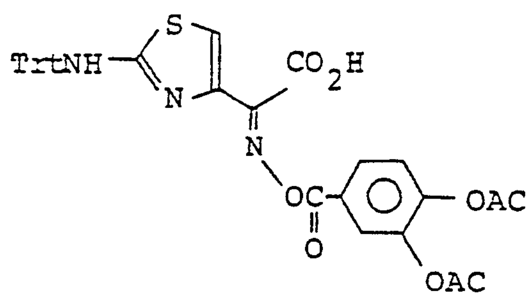

It is prepared according to the following steps 1) - 3):

1) Preparation of diphenylmethyl 2-(2-tritylaminothiazole-4-yl)-2-syn-hydroxyiminoacetate:

At first, 8.58 g of 2-(2-tritylaminothiazole-4-yl)-2-syn-hydroxyiminoacetic acid is suspended in 100 ml of tetrahydrofuran, and then diphenyldiazomethane is added to the suspension at room temperature with stirring until the red color of the reaction mixture becomes not to disappear. Then, 1 ml of acetic acid is added to the mixture, and the mixture is diluted with ethyl acetate. The organic layer is washed with a saturated aqueous sodium chloride solution, a saturated aqueous sodium bicarbonate solution and a saturated aqueous sodium chloride solution in this order, dried over sodium sulfate, and concentrated under reduced pressure to obtain a crude ester compound. The resulting ester compound is purified by silica gel column chromatography [Wako gel C-100, made by Wako Junyaku Co.; developing system n-hexane : ethyl acetate = 3 : 1 V/V) to obtain 6.07 g of ester compound in a yield of 51 %.

NMR ($CDCl_3$)δ: 7.13(25H, m), 7.07(1H, s), 6.10(1H, s)

2) Preparation of diphenylmethyl 2-(2-tritylaminothiazole-4-yl)-2-syn-(3,4-diacetoxybenzoyloxyimino)acetate:

At first, 910 mg of diphenylmethyl 2-(2-tritylaminothiazole-4-yl)-2-syn-hydroxyiminoacetate is dissolved in 10 ml of anhydrous methylene chloride, and 0.230 ml of triethylamine is added to the solution with ice cooling and stirring. Then, an acid chloride which is obtained by suspending 385.6 mg of 3,4-diacetoxybenzoic acid in 3 ml of anhydrous methylene chloride, adding 337 mg of phosphorus pentachloride thereto with stirring to subject the resulting mixture to reaction for 30 minutes, and then concentrating the reaction mixture under reduced pressure, is added to the solution. The mixture is subjected to reaction for one hour, diluted with 20 ml of chloroform, washed with a saturated aqueous sodium bicarbonate solution and a saturated aqueous sodium chloride solution in this order, dried over sodium sulfate, and concentrated under reduced pressure to obtain 1.13 g of 3,4-diacetoxybenzoyl compound.

NMR ($CDCl_3$)δ: 7.5-7.1(27H, m), 7.0(1H, d, J = 9Hz), 6.80(1H, s), 2.30(3H, s), 2.27(3H, s)

3) Preparation of 2-(2-tritylaminothiazole-4-yl)-2-syn-(3,4-diacetoxybenzoyloxyimino) acetic acid:

At first, 1.13 g of diphenylmethyl 2-(2-tritylaminothiazole-4-yl)-2-syn-(3,4-diacetoxybenzoyloxyimino) acetate is dissolved in 5 ml of anhydrous methylene chloride and 5 ml of anhydrous nitromethane, and 880 mg of aluminum chloride dissolved in 4 ml of anhydrous nitromethane is added dropwise to the solution with ice cooling and stirring. Then, 20 minutes after the dropwise addition, the reaction mixture is diluted with 50 ml of ethyl acetate, washed once with 1 N hydrochloric acid and twice with a saturated aqueous sodium chloride solution, dried over sodium sulfate, and concentrated under reduced pressure. Then, 20 ml of toluene is added to the residue, and the resulting mixture

is disintegrated. The toluene layer is removed by decantation to obtain 649 mg of carboxylic acid. An equal amount of n-hexane is added to the toluene layer, and the deposited precipitates are collected to further obtain 100 mg of carboxylic acid.

NMR (DMSO-$d_6$)δ: 7.9-7.8(2H, m), 7.6-7.1(17H, m), 2.27(6H, s)

IR $\nu_{max}^{cm^{-1}}$ (KBr) : 3435, 1790, 1775(sh.), 1750(sh.), 1600

<u>Example 2</u>

Preparation of 2-(2-tritylaminothiazole-4-yl)-2-syn-(3,4-diacetoxybenzoyloxyimino) acetic acid (another process):

At first, 4.29 g of 2-(2-tritylaminothiazole-4-yl)-2-syn-hydroxyiminoacetic acid is suspended in 50 ml of anhydrous methylene chloride, and 2.77 ml of triethylamine is added to the suspension with ice cooling and stirring to dissolve the acid. Then, an acid chloride, which is prepared from 2.34 g of 3,4-diacetoxybenzoic acid in the same manner as in Example 1 - 2), is dissolved in 10 ml of anhydrous methylene chloride, and the solution is added to the mixture dropwise. One hour after the dropwise addition, the reaction mixture is washed once with 1 N hydrochloric acid and twice with a saturated aqueous sodium chloride solution, dried over sodium sulfate and concentrated under reduced pressure to obtain crude carboxylic acid. Ether is added to the residue. The residue is disintegrated, stirred in ether overnight, and separated by filtration to obtain 5.31 g of carboxylic acid. IR and NMR of the present compound are identical with those obtained in Example 1 - 3).

<u>Example 3</u>

Preparation of 2-(2-formylamino-thiazole-4-yl)-2-syn-(3,4-diacetoxybenzoyloxyimino) acetic acid:

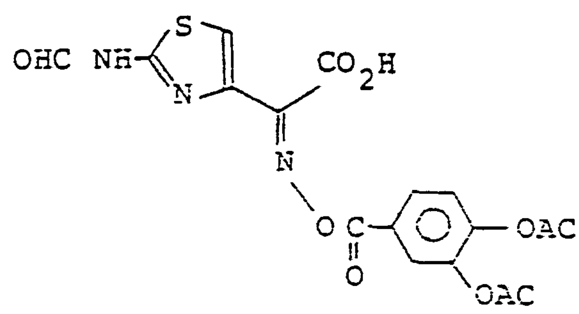

At first, 215 mg (1 millimole) of 2-(2-formylaminothiazole-4-yl)-2-syn-hydroxyiminoacetic acid and 306 μl (2.2 millimoles) of triethylamine are dissolved in 4 ml of dichloromethane, and the solution of 256 mg (1 millimole) of 3,4-diacetoxybenzoyl chloride in 3 ml of dichloromethane is added to the solution with ice cooling and stirring. After stirring with ice cooling for 30 minutes and successive stirring at room temperature for 30 minutes, the reaction mixture is poured into water, and the resulting mixture is adjusted to pH 1 with 10% hydrochloric acid. Then, the mixture is extracted twice with ethyl acetate. The extract is washed with an aqueous sodium chloride solution, dried over sodium sulfate, and concentrated under reduced pressure. The resulting residue is disinteglated in ethyl acetate-hexane (1 : 1) and recrystallized from chloroform-methanol to obtain 150 mg of the desired compound.

NMR ($CD_3OD$) δ: 8.57(1H, s), 8.2-7.7(2H, m), 7.5-7.3 (2H, m), 3.3(6H, s)

IR $\nu_{max}^{cm^{-1}}$ (KBr): 3150(br), 1795, 1782, 1775, 1750, 1559

Example 4

Preparation of (6R, 7S)-7-[2-(2-aminothiazole-4-yl)-2-syn-(3,4-diacetoxybenzoyloxyimino)-acetamido-1-azabicyclo[4,2,0]-oct-2-en-8-oxo-2-carboxylic acid:

BAD ORIGINAL

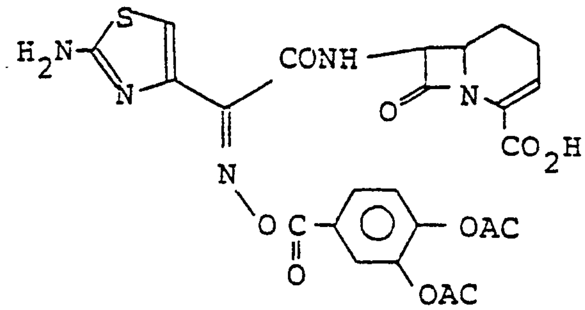

At first, 1.23 g of 2-(2-trityl-aminothiazole-4-yl)-2-syn-(3,4-diacetoxybenzoyloxyimino) acetic acid is suspended in 20 ml of anhydrous methylene chloride, and 367 mg of phosphorus pentachloride is added to the suspension with cooling to -30°C and stirring. Temperature of the mixture is returned to room temperature over 30 minutes, and the mixture is concentrated under reduced pressure. Then, 30 ml of anhydrous tetrahydrofuran is added to the residue to prepare an acid chloride solution.

Separately, 325 mg of (6R, 7S)-7-amino-1-azabicyclo [4,2,0]octo-2-en-8-oxo-2-carboxylic acid is suspended in 15 ml of tetrahydrofuran and 15 ml of water with ice cooling and stirring, and the suspension is adjusted to pH 8.0 with triethylamine to dissolve the carboxylic acid. The said acid chloride solution is added dropwise to the solution while keeping pH at 7.5 - 8.5 with triethylamine. After reaction for 30 minutes from the dropwise addition, pH is adjusted to 2.0 with 1 N hydrochloric acid, ethyl acetate is added to the reaction mixture, and the separated organic layer is recovered. The aqueous layer is extracted twice with ethyl acetate, and the organic layers are joined together, dried over sodium sulfate, and concentrated under reduced pressure. Ether is added to the residue, and the residue is disintegrated and stirred in ether for 30 minutes. The ether is removed by decantation, 50 ml of 50% acetic acid is added to the remaining mixture, and the resulting mixture is stirred with heating to 60°C for 30 minutes. Then, the resulting mixture is concentrated under reduced pressure, and 40 ml of ether and 20 ml of ethyl acetate are added to

BAD ORIGINAL

0075805

the residues. After stirring for 30 minutes, the organic layer is removed by decantation, and the residue is dissolved in dimethylsulfoxide. The solution is charged onto a column chromatograph of 25 ml of Diaion HP-10, made by Mitsubishi Kasei Kogyo Co.

After water washing, elution with a gradient from water to water / methanol [ = 1 : 4 (V/V) ] is carried out to obtain 322 mg of acyl compound.

NMR ($DMSO-d_6$ + $CD_3OD$)δ: 8.15-7.95(2H, m), 7.43(1H, d, J = 9Hz), 7.20(1H, s), 6.37(1H, m), 5.63(1H, d, J = 4.5Hz), 4.10-3.30(1H, m), 2.30(6H, s), 2.50-1.40(4H, m)

IR $\nu_{max}^{cm^{-1}}$ (KBr): 3450, 1780, 1750(sh), 1730(sh), 1670, 1640, 1545

Example 5

Preparation of (6R, 7S)-7-[2-(2-aminothiazole-4-yl)-2-syn-(3,4-diacetoxybenzoyloxyimino) acetamido]-1-azabicyclo[4,2,0]oct-2-en-8-oxo-2-carbocylic acid (another process):

At first, 130 mg (0.30 millimoles) of 2-(2-formylaminothiazole-4-yl)-2-syn-(3,4-diacetoxybenzoyloxyimino) acetic acid is dissolved in 3 ml of anhydrous tetrahydrofuran, and 50 μl (0.36 millimoles) of triethylamine is added to the solution with cooling by dry ice-carbon tetrachloride. Then, 62 mg (0.30 millimoles) of phosphorus pentachloride is added to the solution, which is then further stirred at the same temperature for one hour to prepare an acid chloride-tetrahydrofuran solution.

Separately, 30 mg (0.15 millimoles) of (6R, 7S)-7-amino-1-azabicyclo[4,2,0]octo-2-en-8-oxo-2-carboxylic acid is dissolved in 1.5 ml of water and 0.8 ml of tetrahydrofuran, and the solution is adjusted to pH 7.8 with triethylamine with ice cooling. Then, the said acid chloride solution is added thereto dropwise with ice cooling while keeping pH at 7.5 - 8.0 with triethylamine, and the mixture is further stirred at 0°C for 30 minutes. Then, 15 ml of water is added

to the mixture, which is then washed with ethyl acetate and adjusted to pH 2.0 with 1.0 N hydrochloric acid. The mixture is extracted twice with ethyl acetate / tetrahydrofuran (3 / 1). The extract is washed with an aqueous sodium chloride solution, dried over sodium sulfate, and then concentrated under reduced pressure. The residue is disintegrated in ethyl acetate, and the precipitate is separated by filtration and dried to obtain 45 mg of (6R, 7S)-7-[2-(2-formylaminothiazole-4-yl)-2-syn-(3,4-diazetoxybenzoyloxyimino) acetamido-1-azabicyclo[4,2,0]oct-2-en-8-oxo-2-carboxylic acid.

Then, 40 mg of the formyl compound thus obtained is mixed with a mixture of 2 ml of methanol and 0.15 g of concentrated hydrochloric acid, and the resulting mixture is stirred at room temperature for one hour. The resulting solution is adjusted to pH 6.0 with an aqueous $NaHCO_3$ solution, and then concentrated under reduced pressure. The residue is adjusted to pH 2.0, and purified by Diaion HP-10 (water ∿ water : methanol = 1 : 8) to obtain 19 mg of the desired compound. Physical properties are quite identical with those obtained in the process of Example 4.

Example 6

Preparation of (6R, 7S)-7-[2-(2-aminothiazole-4-yl)-2-syn-(3,4-diacetoxybenzoyloxyimino) acetamido]-1-azabicyclo[4,2,0]oct-2-en-8-oxo-2-carboxylic acid (another process):

It is prepared according to the following steps 1) - 3):

1) Preparation of diphenylmethyl (6R, 7S)-7-[2-(2-trytylaminothiazole-4-yl)-2-syn-hydroxyiminoacetamido]-1-azabicyclo[4,2,0]oct-2-en-8-oxo-2-carboxylate:

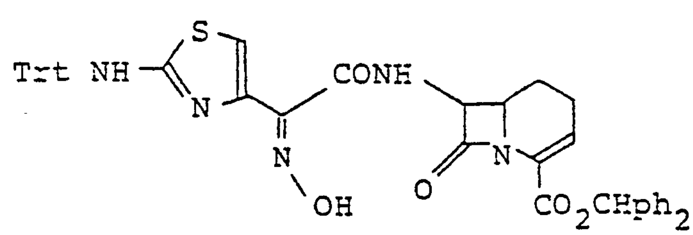

At first, 6.3 g of 2-(2-tritylaminothiazole-4-yl)-2-syn-(1-methoxy-1-methyl)ethoxyiminoacetic acid is dissolved in 30 ml of anhydrous tetrahydrofuran, and then 1.7 ml of triethylamine is added thereto with cooling to -20°C and stirring. Then, 2.57 g of phosphorus pentachloride is added to the mixture to carry out reaction for 30 minutes, whereby an acid chloride solution is prepared.

Separately, 1.5 g of (6R, 7S)-7-amino-1-azabicyclo [4,2,0]oct-2-en-8-oxo-2-carboxylic acid is suspended in 50 ml of tetrahydrofuran and 50 ml of water, and the suspension is adjusted to pH 8.0 with triethylamine with ice cooling and stirring, whereby the carboxylic acid is dissolved. Then, the said acid chloride solution is added dropwise to the solution, while keeping the solution at pH 7.5 - 9.0 with triethylamine, and 30 minutes after the dropwise addition, sodium chloride is added thereto to saturation. The mixture is adjusted to pH 2.0 with 1 N hydrochloric acid, and the separated organic layer is recovered. The aqueous layer is extracted twice with a mixed solvent of tetrahydrofuran and ethyl acetate in equal amounts. The organic layers are joined together, washed with a saturated aqueous sodium chloride solution, dried over sodium sulfate and concentrated under reduced pressure, whereby 10.3 g of crude acyl compound is obtained.

Then, 50 ml of acetone and 50 ml of 1 N hydrochloric acid are added to 10.3 g of the crude acyl compound as such to dissolve it, and the solution is allow to stand for 40 minutes. Then, 50 ml of water is added thereto, and after only acetone is distilled off under reduced pressure, the mixture is extracted twice with a mixed solvent of tetrahydrofuran and ethyl acetate in equal amounts. The organic layers are joined together, dried over sodium sulfate, and concentrated to obtain 7.32 g of crude hydroxyiminoacyl compound.

Then, 7.32 g of the crude hydroxyiminoacyl compound is dissolved in 50 ml of tetrahydrofuran and 50 ml of ethyl acetate, and diphenyldiazomethane is added thereto until the red color of the reaction mixture become not to disappear.

0075805

Then, the reaction mixture is concentrated under reduced pressure, and the residue is washed three times with n-hexane and purified by silica gel column chromatography (Wako gel C-200 : 100 g, solvent n-hexane-ethyl acetate 3 : 1 V/V) to obtain 837 mg of the desired diphenylmethyl ester compound.

NMR ($CD_3Cl$)δ: 7.6-7.0(25H, m), 6.93(1H, s), 6.75(1H, s), 6.46(1H, m), 5.30(1H, d-d, J = 6, 6Hz), 3.80(1H, m), 2.4-1.3(4H, m)

2) Preparation of diphenylmethyl (6R, 7S)-7-[2-(2-tritylaminothiazole-4-yl)-2-syn-(3,4-diacetoxybenzoyloxyimino) acetamido]-1-azabicyclo[4,2,0]oct-2-en-8-oxo-2-carboxylate:

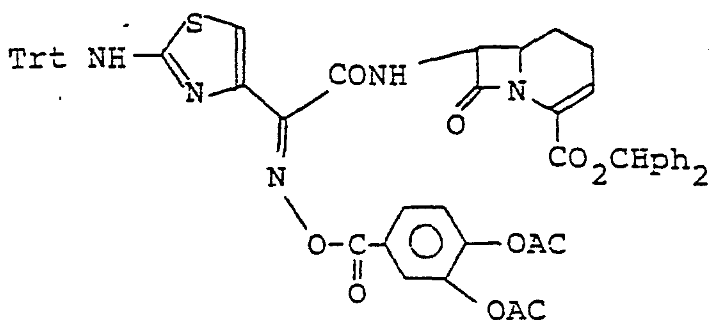

At first, 151.8 mg of diphenylmethyl (6R, 7S)-7-[2-(2-tritylaminothiazole-4-yl)-2-syn-hydroxyiminoacetamido]-1-azabicyclo[4,2,0]oct-2-en-8-oxo-2-carboxylate prepared in Example 6 - 1) is dissolved in 3 ml of anhydrous methylene chloride, and then 30 μl of triethylamine is added thereto with ice cooling and stirring. An acid chloride prepared from 57.1 mg of 3,4-diacetoxybenzoic acid is dissolved in 2 ml of methylene chloride, and the solution is added to the said mixture to carry out reaction for one hour. Then, the reaction mixture is washed three times with water, dried over sodium sulfate, and concentrated under reduced pressure. The residue is purified by silica gel column chromatography (silica gel 20 g, n-hexane-ethyl acetate 2 : 1 V/V) to obtain 151 mg of the desired benzoyl compound.

NMR ($CDCl_3$) δ: 8.0-7.8(7H, m), 7.6-7.0(26H, m), 6.90(1H, s), 6.70(1H, s), 6.43(1H, m), 5.50(1H, d-d, J = 9, 5Hz), 2.5-1.0(4H, m), 2.27(6H, s)

3) Preparation of (6R, 7S)-7-[2-(2-aminothiazole-4-yl)-2-syn-(3,4-diacetoxybenzoylcarbonyloxyimino) acetamido]-1-azabicyclo[4,2,0]oct-2-en-8-oxo-2-carboxylic acid:

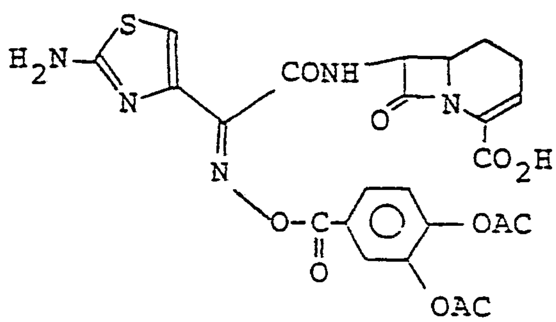

At first, 151 mg of diphenylmethyl (6R, 7S)-7-[2-(2-tritylaminothiazole-4-yl)-2-syn-(3,4-diacetoxybenzoyloxyimino) acetamido]-1-azabicyclo[4,2,0]oct-2-en-8-oxo-2-carboxylate prepared in Example 6 - 2) is dissolved in 1 ml of anhydrous methylene chloride and 0.1 ml of anisol, and then 1 ml of trifluoroacetic acid is added thereto. The mixture is allowed to stand at room temperature for one hour, and then concentrated under reduced pressure. Ethyl acetates is again added to the residue, and after concentration in reduced pressure, 2 ml of an aqueous 50% acetic acid solution is added to the residue. Then, the mixture is heated to 50°C with stirring for one hour, and then concentrated under reduced pressure. The residue is washed with ether, dissolved in dimethylformamide, purified by silica gel chromatography (silica gel 5 g, ethyl acetate : acetic acid = 10 : 1 V/V), and disintegrated twice in ether to obtain 41 mg of carboxylic acid. The results of both NMR and IR of the compound are identical with those obtained in Example 4.

BAD ORIGINAL

Example 7

Preparation of (4S, 6R, 7S)-7-[2-(2-aminothiazole-4-yl)-2-syn-(3,4-diacetoxybenzoyloxyimino) acetamido]-1-azabicyclo[4,2,0]oct-2-en-8-oxo-2-carboxylic acid:

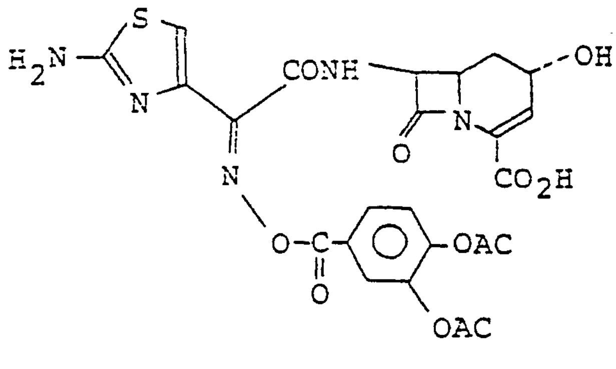

At first, 649 mg of 2-(2-tritylaminothiazole-4-yl)-2-syn-(3,4-diacetoxybenzoyloxyimino) acetic acid is suspended in 4 ml of anhydrous tetrahydrofuran, and then 151 µl of triethylamine and 208 mg of phosphorus pentachloride are added thereto with cooling to -20°C and stirring to carry out reaction for 30 minutes, whereby an acid chloride solution is obtained.

Separately, 100 mg of (4S, 6R, 7S)-7-amino-1-azabicyclo[4,2,0]-oct-2-en-4-hydroxy-8-oxo-2-carboxylic acid is suspended in 10 ml of tetrahydrofuran and 10 ml of water, and then dissolved by adding triethylamine thereto with ice cooling and stirring to make pH 8.0. Then, the said acid chloride solution is added thereto dropwise with ice cooling and stirring while keeping pH 7.5 - 9.0 with triethylamine. The mixture is subjected to reaction for 30 minutes after the dropwise addition, and adjusted to pH 2.0 with 1 N hydrochloric acid. Ethyl acetate is added thereto and the separated organic layer is recovered. The aqueous layer is extracted twice with ethyl acetate, and the organic layers are joined together, washed with a saturated aqueous sodium chloride solution, dried over sodium sulfate and concentrated under reduced pressure. Ether is added to the residue, which is then disintegrated, and the ether layer is removed by decantation to obtain a crude acyl compound. Then, 50 ml

of an aqueous 50% acetic acid solution and 5 ml of methanol are added to the crude compound, and the mixture is heated at 50°C with stirring for one hour, and then concentrated under reduced pressure. The residue is subjected to distribution with water and ethyl acetate, and the ethyl acetate layer is extracted again with water. The aqueous layers are joined together, washed with ethyl acetate, and concentrated under reduced pressure. The residue is purified with 20 ml of Diaion HP-10, and a fraction eluted by methanol containing 33% of water is recovered, whereby 35 mg of acyl compound is obtained.

NMR ($CD_3OD$) δ: 8.0-7.7(2H, m), 7.6-7.3(1H, m), 7.30(1H, s), 6.27(1H, d, J = 6Hz), 5.63(1H, d, J = 5Hz), 2.30(6H, s), 2.2-1.5(2H, m)

IR $\nu_{max}^{cm^{-1}}$ (KBr): 3550, 3400, 3330, 1780, 1760, 1660, 1620

Example 8

Preparation of 7-[2-(2-aminothiazole-4-yl)-2-syn-(3,4-diacetoxybenzoyloxyimino)acetamido]-3-(1-methyl-1H-tetrazole-5-yl) thiomethyl-3-cephem-4-carboxylic acid:

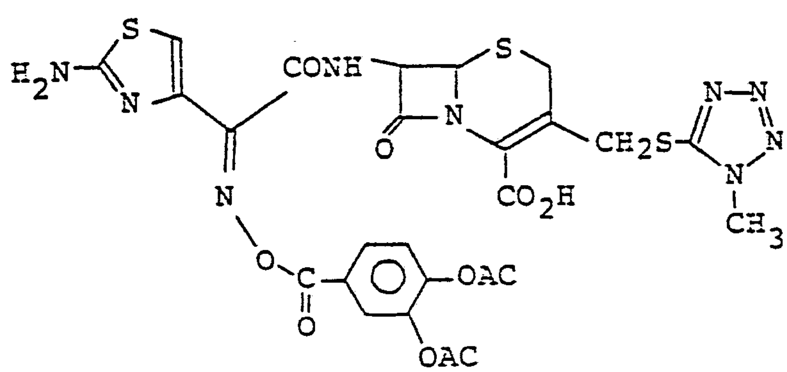

At first, 700 mg of 2-(2-trytylaminothiazole-4-yl)-2-syn-(3,4-diacetoxybenzoyloxyimino) acetic acid is suspended in 6 ml of anhydrous tetrahydrofuran, and 111 µl of triethylamine and 208 mg of phosphorus pentachloride are added to the suspension with cooling to -20°C and stirring. The mixture

BAD ORIGINAL

is subjected to reaction for 30 minutes to prepare an acid chloride solution.

Separately, 185 mg of 7-amino-3-(1-methyl-1H-tetrazole-5-yl)-thiomethyl-3-cephem-4-carboxylic acid is suspended in 7 ml of tetrahydrofuran and 7 ml of water, and the mixture is adjusted to pH 8.0 with triethylamine with ice cooling and stirring to dissolve the carboxylic acid. Then, the said acid chloride solution is added thereto dropwise, while maintaining the mixture to pH 7.5 - 9.0 with triethylamine. After reaction for 30 minutes from the dropwise addition, the reaction mixture is adjusted to pH 2.0 with 1 N hydrochloric acid, ethyl acetate is added to the mixture, and the separated organic layer is recovered. The aqueous layer is extracted twice with ethyl acetate. The organic layers are joined together, washed with a saturated aqueous sodium chloride solution, dried over sodium sulfate, and then concentrated under reduced pressure. Ether is added to the residue, which are then disintegrated, and the ether is removed by decantation. Then, 70 ml of an aqueous 50% acetic acid solution is added to the resulting crude acyl compound, and the mixture is heated at 50°C with stirring for 30 minutes.

Then, the mixture is concentrated under reduced pressure, and the residue is washed with a mixed solvent of ether and ethyl acetate in equal amounts and dissolved in dimethylsulfoxide. The solution is charged onto 15 ml of Diaion HP-10, and subjected to elution with a gradient from water to water / methanol ( = 5/1 V/V ) to obtain 35 mg of acyl compound. The acyl compound is further purified with 10 ml of Diaion HP-10 in the same manner as above to obtain 19 mg of desired acyl compound.

NMR ($CD_3OD$)δ: 8.0-7.7(2H, m), 7.4-7.2(2H, m), 5.87(1H, d, J = 4.5Hz), 5.15(1H, d, J = 4.5Hz), 3.97(3H, s), 2.28(6H, s)

IR $\nu_{max}^{cm^{-1}}$ (KBr): 3430, 1780, 1750(sh), 1650

BAD ORIGINAL

Ether is added to 19 mg of the resulting acyl compound, and the mixture is fractured. Then, the ether is removed therefrom by decantation. Ether is again added to the residue, and the mixture is stirred for washing. Then, the ether is removed therefrom by decantation. The residue is dried under reduced pressure to obtain 12 mg of purified acyl compound.

NMR δ($DMSOd_6$ + $CD_3OD$): 8.05 - 7.90(1H, m), 7.95(1H, s), 7.44(1H, d, J = 8.1Hz), 7.17(1H, s), 5.92(1H, d, J = 5.0Hz), 5.19(1H, d, J = 5.0Hz), 4.46(1H, d, J = 13.4Hz), 4.25(1H, d, J = 13.4Hz), 3.94(3H, s), 3.73(1H, s), 3.70(1H, s), 2.31(3H, s), 2.29(3H, s)

IR $\nu_{max}^{KBr}$ ($cm^{-1}$): 3450, 3350, 2950, 1780, 1680, 1635, 1615

0075805

Example 9

Preparation of triethylamine salt of 2-(2-tritylaminothiazole-4-yl)-2-syn-(3,4-bisdihydroxypyranyloxyphenyl-1-yl) methoxyiminoacetic acid:

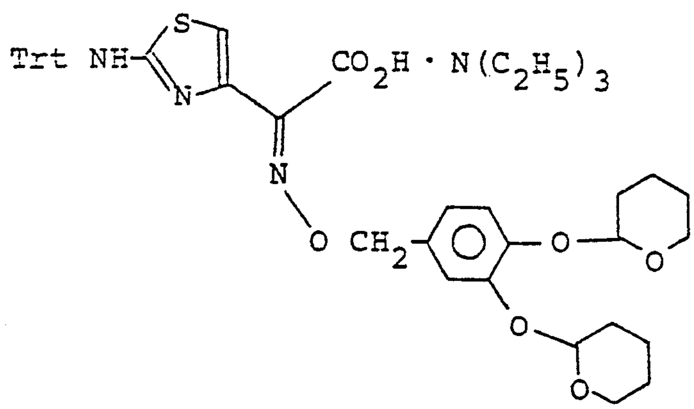

It is prepared according to the following steps 1) and 2).

1) Preparation of ethyl 2-(2-tritylaminothiazole-4-yl)-2-syn-(3,4-bisdihydroxypyranyloxyphenyl-1-yl) methoxyiminoacetate:

At first, 3.25 g of ethyl 2-(2-tritylaminothiazole-4-yl)-2-syn-hydroxyiminoacetate is dissolved in 20 ml of anhydrous dimethylformamide, and 10 ml of anhydrous ether is added thereto. Then, 430 mg of 60% oil-dispersed sodium hydride is added thereto with ice cooling and stirring. 3,4-Bisdihydropyranyloxybenzyl chloride, which is prepared from 2.84 g of 3,4-bisdihydropyranyloxybenzyl alcohol prepared in Reference Example 2 and dissolved in 10 ml of dimethylsulfoxide, is added thereto. The mixture is brought back to room temperature, and stirred overnight.

Then, the mixture is diluted with ethyl acetate, washed 5 times with water and once with a saturated aqueous sodium bicarbonate solution in this order, dried over sodium sulfate, and then concentrated under reduced pressure. The residue is purified by silica gel column chromatography (Wako gel C-200 200 g, n-hexane-ethyl acetate = 3 : 1 V/V) to obtain 1.15 g of substituted oxyimino compound.

NMR ($CDCl_3$)δ: 7.5-6.8(18H, m), 6.50(1H, s), 5.40(2H, m), 5.17(2H, s), 4.32(2H, q, J = 6.6Hz), 4.2-3.5(4H, m), 2.2-1.3(12H, m), 1.23(3H, q, J = 6.6Hz)

2) At first, 1.15 g of ethyl 2-(2-tritylaminothiazole-4-yl)-2-syn-(3,4-bisdihydroxypyranyloxyphenyl-1-yl) methoxyiminoacetate is dissolved in 50 ml of dioxane, 2.17 ml of an aqueous 1 N sodium hydroxide solution is added thereto, and the mixture is refluxed with stirring. Then, 1 ml of the aqueous sodium hydroxide solution is added thereto after 2 hours from the start of reflux, 1 ml thereof after 6 hours, 1 ml thereof after 8 hours, 1 ml thereof after 10 hours, and 2 ml thereof after being allowed to stand overnight. Then, reflux with stirring is again started, and 2 ml thereof is added thereto after 6 hours. Then, the reaction mixture is further refluxed for one hour and concentrated under reduced pressure, and water is added to the residue. The mixture is adjusted to pH 2.0 with 1 N hydrochloric acid with ice cooling and stirring, and then extracted twice with ethyl acetate. The ethyl acetate layer is washed with a saturated sodium chloride solution, dried over sodium sulfate, and filtered. Then, 1 ml of triethylamine is added to the filtrate, and the mixture is concentrated under reduced pressure to obtain 1.24 g of the desired triethylamine salt of carboxylic acid. The salt thus obtained is used as such in acylation of the following Example 10.

Example 10

Preparation of sodium salt of (6R, 7S)-7-[2-(2-aminothiazole-4-yl)-2-syn-(3,4-dihydroxyphenyl-1-yl) methoxyimino) acetamido]-1-azabicyclo[4,2,0]oct-2-en-8-oxo-2-carboxylic acid:

BAD ORIGINAL

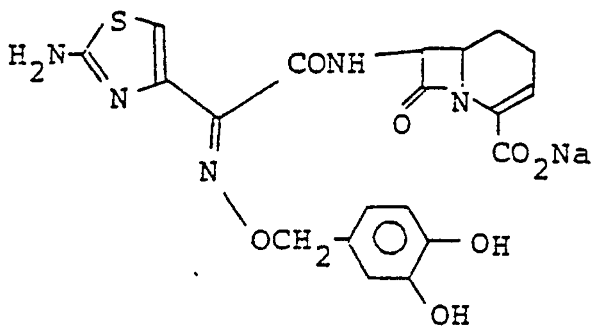

At first, 620 mg of triethylamine salt of 2-(2-tritylaminothiazole-4-yl)-2-syn-(3,4-bisdihydroxypyranyloxy-phenyl-1-yl) methoxyimino acetic acid prepared in Example 9 is dissolved in 4 ml of anhydrous tetrahydrofuran, and 104 mg of phosphorus pentachloride is added thereto with cooling to -20°C and stirring. The mixture is subjected to reaction for 30 minutes to prepare an acid chloride solution.

Separately, 80 mg of (6R, 7S)-7-amino-1-azabicyclo [4,2,0]oct-2-en-8-oxo-2-carboxylic acid is suspended in 10 ml of tetrahydrofuran and 10 ml of water, and the suspension is adjusted to pH 8.0 with triethylamine with ice cooling and stirring to dissolve the carboxylic acid. Then, the said acid chloride solution is added dropwise to the solution while keeping pH 7.5 - 9.0 with triethylamine. The mixture is subjected to reaction for 30 minutes after the dropwise addition, and an acid chloride solution prepared from 650 mg of triethylamine salt of the carboxylic acid in the same manner as above is added to the reaction mixture, and the mixture is further subjected to reaction for 30 minutes. Then, the mixture is adjusted to pH 2.0 with 1 N hydrochloric acid, and ethyl acetate is added thereto. The resulting two layers are separated, and the aqueous layer is extracted twice with ethyl acetate. The organic layers are joined together, washed with a saturated aqueous sodium chloride solution, dried over sodium sulfate, and concentrated under reduced pressure. The residue is disintegrated in ether, and the ether is removed by decantation. The resulting powder is washed twice with ether and dried.

BAD ORIGINAL

0075805

Then, 10 ml of 50% acetic acid is added thereto, and the mixture is heated to 60°C with stirring for one hour and 30 minutes and then concentrated under reduced pressure. Then, 2 ml of a saturated aqueous sodium bicarbonate solution is added to the residue to dissolve it. The solution is washed twice with ethyl acetate and charged onto 15 ml of Diaion HP-10, and the fraction eluted with an aqueous 25% methanol solution is recovered, whereby 23.1 mg of the desired acyl compound is obtained.

NMR ($D_2O$)δ: 7.03(4H, m), 6.27(1H, t, J = 3Hz), 5.47 (1H, d, J = 4.5Hz), 5.15(2H, brs), 2.5 - 1.5(4H, m)

IR $\nu_{max}^{cm^{-1}}$ (KBr): 3300, 1770(sh), 1755, 1750(sh)

Example 11

Preparation of 7-[2-(2-aminothiazol-4-yl)-2-syn-(3,4-diacetoxybenzoyloxyimino) acetamido]-3-acetoxymethyl-3-cephem-4-carboxylic acid:

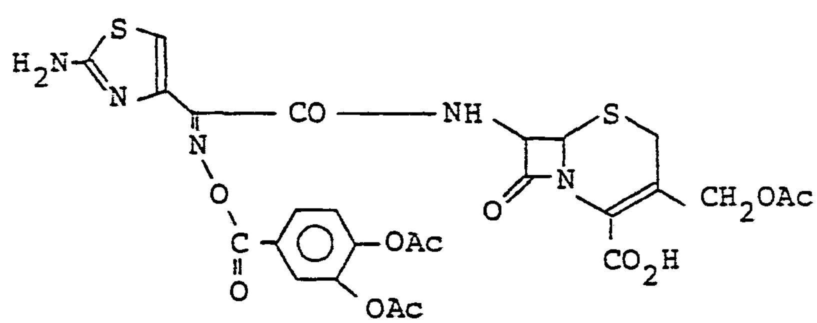

At first, 145 mg of 2-(2-tritylamino-4-thiazolyl)-2-syn-(3,4-diacetoxybenzoyloxyimino) acetic acid is dissolved in 5 ml of anhydrous tetrahydrofuran, and 32.5 μl of triethylamine and 49.5 mg of phosphorus pentachloride are added thereto with cooling to -30°C and stirring. Then, the mixture is subjected to reaction with ice cooling and stirring for one hour to prepare an acid chloride solution.

Separately, 97.2 mg of 7-amino-3-acetoxymethyl-3-cephem-4-carboxylic acid is suspended in 5 ml of tetrahydrofuran and 5 ml of water, and the suspension is adjusted to pH 9.0 with triethylamine with ice cooling and stirring to dissolve the amino acid. The said acid chloride solution is added dropwise to the amino acid solution, while adding triethylamine thereto to keep the pH of the reaction mixture at 7.5 - 9.0. The mixture is subjected to reaction for 30 minutes after the dropwise addition, adjusted to pH 2.0 with 1 N hydrochloric acid, and extracted three times with ethyl acetate. The organic layers are joined together, washed twice with a saturated aqueous sodium chloride solution, dried over anhydrous sodium sulfate, and concentrated under reduced pressures. Then, 20 ml of an aqueous 50% acetic acid solution is added to the residue, and the mixture is heated at 60°C with stirring for one hour and concentrated under reduced pressure. Then, the residue is disintegrated in 20 ml of ether, and the ether layer is removed by decantation to obtain a crude acyl compound. The crude acyl compound is dissolved in a small amount of dimethylsulfoxide, charged onto a column of 12 ml of Diaion HP-10, and subjected to elution with a gradient from water to water-methanol (1/5 V/V), to obtain 27 mg of acyl compound.

IR $\nu_{max}^{KBr}$ ($cm^{-1}$): 3350, 1780, 1750(sh), 1680, 1670, 1615, 1540

NMR(DMSOd6 + $CD_3OD$)$\delta$: 8.2 - 7.8(2H, m), 7.6 - 7.2(1H, m), 7.16(1H, s), 5.93(1H, d, J = 4.8Hz), 5.19(1H, d, J = 4.8Hz), 5.07(1H, d, J = 13.2Hz), 4.74(1H, d, J = 13.2Hz), 3.72(1H, d, J = 20Hz), 3.40(1H, d, J = 20Hz), 2.30(6H, s), 2.04(3H, s)

Example 12

Preparation of (4S, 6R, 7S)-7-[2-(2-aminothiazol-4-yl)-2-syn-(3,4-diacetoxybenzoyloxyimino) acetamido]-4-methoxy-1-azabicyclo [4,2,0] oct-2-en-8-oxo-2-carboxylic acid:

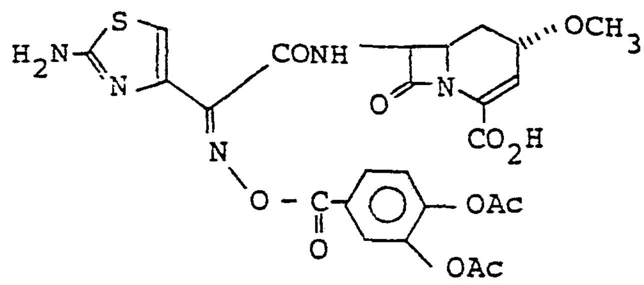

At first, 300 mg of 2-(2-tritylaminothiazol-4-yl)-2-syn-(3,4-diacetoxybenzoyloxyimino) acetic acid is dissolved in 4 ml of anhydrous tetrahydrofuran, and 71 μl of triethylamine is added thereto with cooling to -20°C. Then, 110 mg of phosphorus pentachloride is added thereto, and the mixture is stirred with ice cooling for one hour to prepare an acid chloride solution.

Separately, 107 mg of (4S, 6R, 7S)-7-amino-4-methoxy-1-azabicyclo [4,2,0] oct-2-en-8-oxo-2-carboxylic acid prepared in Reference Example 4 is dissolved in a mixed solvent of 6 ml of water and 6 ml of tetrahydrofuran, and while triethylamine is added thereto with ice cooling and stirring to keep the pH of the solution at 7.5 - 8.0, the said acid chloride solution is added thereto dropwise. Then, the mixture is stirred at the same temperature for one hour, adjusted to pH 2 with 0.5 N hydrochloric acid with ice cooling and extracted twice with ethyl acetate. The organic layers are washed once with a saturated sodium chloride solution and dried over sodium sulfate. The solvent is distilled off under reduced pressure. Then, 10 ml of an aqueous 50% acetic acid solution is added to the residue, and the mixture is stirred at 50°C for 45 minutes. The reaction mixture is concentrated, and the residue is thoroughly disintegrated in ether. Solid matters are separated by filtration, dissolved in a small amount of dimethylsulfoxide, and adsorbed on 20 ml of Diaion HP-10 in a column. A fraction containing the desired compound, which is eluted with water-methanol (2 : 3), is concentrated under reduced pressure to obtain 94 mg of crude powder. The crude powder is disintegrated in a mixture of 2 ml of ethyl acetate and

2 ml of ether, and the solid matters are separated by filtration to obtain 78 g of desired compound as powder. Yield 25.8%.

NMR($CD_3OD$)δ: 1.4 - 2.4(2H, m), 2.31(6H, brs), 3.33(3H, s), 3.84 - 4.16(2H, m), 5.72(1H, d, J = 5.5Hz), 6.42(1H, d, J = 5.4Hz), 7.29(1H, s), 7.37 - 8.05 (3H, m)

IR $\nu_{max}^{KBr}$ ($cm^{-1}$): 1790(sh), 1780, 1770, 1760(sh), 1620 - 1690

Example 13

Preparation of 2-(2-tritylaminothiazol-4-yl)-2-syn-(3,4-diacetoxybenzoylmethoxyimino) acetic acid:

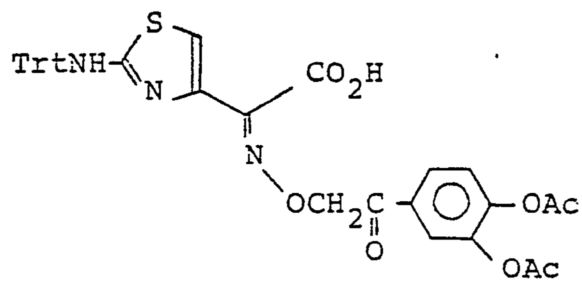

It is prepared according to the following three steps.

13-1) Synthesis of diphenylmethyl 2-(2-tritylaminothiazol-4-yl)-2-syn-(3,4-dihydroxybenzoylmethoxyimino) acetate:

At first, 571 mg of diphenylmethyl 2-(2-tritylaminothiazol-4-yl)-2-syn-hydroxyiminoacetate is dissolved in 5 ml of anhydrous dimethylsulfoxide, and 570 mg of anhydrous potassium carbonate is suspended therein. On the other hand, 373 mg of chloroacetylcatechol is suspended in 10 ml of water, 382 mg of borax is added thereto with stirring, and after the reaction mixture becomes clear, it is concentrated to dryness under reduced pressure. The residue is dried over phosphorus pentoxide under reduced pressure to obtain

a borate of chlcroacetylcatechol is obtained. The borate is then added to the said suspension.

The reaction mixture is stirred at room temperature for 13 hours, then diluted with ethyl acetate, and washed twice with 1 N hydrochloric acid and 5 times with a saturated aqueous sodium chloride solution. The organic layer is dried with anhydrous sodium sulfate and concentrated under reduced pressure. The residue is purified by silica gel chromatography with n-hexane : ethyl acetate = 2 / 1 (V/V) to obtain 506 mg of the desired compound (68%).

NMR $\delta(CDCl_3)$: 7.6 - 6.7(29H, m), 6.20(1H, s), 5.10 (1H, s), 6.27(1H, s), 5,31(2H, s)

13-2) Synthesis of diphenylmethyl 2-(2-tritylaminothiazol-4-yl)-2-syn-(3,4-diacetoxybenzoylmethoxyimino) acetate:

At first, 506 mg of diphenylmethyl 2-(tritylaminothiazol-4-yl)-2-syn-(3,4-dihydroxybenzoylmethoxyimino) acetate is dissolved in 4 ml of pyridine, 0.5 ml of anhydrous acetic acid is added thereto, and the mixture is allowed to stand overnight. Then, the reaction mixture is concentrated under reduced pressure. The residue is diluted with ethyl acetate, washed with 1 N hydrochloric acid and with a saturated aqueous sodium chloride solution, dried over anhydrous sodium sulfate and concentrated under reduced pressure. The residue is purified by silica gel chromatography with n-hexane : ethyl acetate = 2 / 1 (V/V) to obtain 508 mg of the desired compound.

NMR $\delta(CDCl_3)$: 8.9 - 8.6(2H, m), 7.5 - 7.2(25H, m), 7.10(1H, s), 6.23(1H, s), 5.33(2H, s), 2.28(6H, s)

IR $\nu_{max}^{KBr}$ ($cm^{-1}$): 3400, 3070, 3040, 2930, 1785, 1750, 1715, 1610

13-3) Synthesis of 2-(2-tritylaminothiazol-4-yl)-2-syn-(3,4-diacetoxybenzoylmethoxyimino) acetic acid:

At first, 420 mg of diphenylmethyl 2-(2-tritylaminothiazol-4-yl)-2-syn-(3,4-diacetoxybenzoylmethoxyimino) acetate is dissolved in 5 ml of methylene chloride, 5 ml of nitromethane and 0.5 ml of anisole, and then 334 mg of aluminum chloride dissolved in 3 ml of nitromethane is added thereto dropwise with ice cooling and stirring. After the dropwise addition, the mixture is stirred for 5 minutes, diluted with ethyl acetate, washed twice with 1 N hydrochloric acid and further three times with a saturated aqueous sodium chloride solution. The aqueous layers are joined together, and extracted with ethyl acetate. The ethyl acetate layer is washed three times with a saturated aqueous sodium chloride solution, and joined with the former ethyl acetate layer. The joined layers are dried over anhydrous sodium sulfate, and then concentrated under reduced pressure. The residue is washed twice with n-hexane to obtain 319 mg of the desired compound (95%).

NMR $\delta(CDCl_3)$: 8.0 - 7.7(2H, m), 7.5 - 6.8(16H, m), 6.72(1H, s), 5.40(2H, s), 2.30(6H, s)

IR $\nu_{max}^{KBr}(cm^{-1})$: 3450, 3070, 1780, 1715, 1600

<u>Example 14</u>

Preparation of (6R, 7S)-7-[2-(2-aminothiazol-4-yl)-2-syn-(3,4-diacetoxybenzoylmethoxyimino)] acetamido-1-azabicyclo [4,2,0] oct-2-en-8-oxo-2-carboxylic acid:

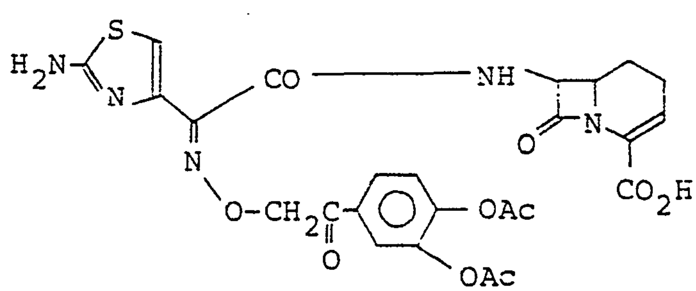

At first, 363 mg of 2-(2-tritylaminothiazol-4-yl)-2-syn-(3,4-diacetoxybenzoylmethoxyimino) acetic acid is dissolved in 5 ml of anhydrous tetrahydrofuran, and 59.4 µl of triethylamine is added thereto. The mixture is cooled to -20°C with stirring. Then, 89.4 mg of phosphorus pentachloride is added thereto, and the mixture is stirred for 30 minutes to prepare an acid chloride solution.

Separately, 182 mg of (6R, 7S)-7-amino-1-azabicyclo [4,2,0]-oct-2-en-8-oxo-2-carboxylic acid is suspended in 5 ml of tetrahydrofuran and 5 ml of water, and dissolved therein by making pH 9.0 with triethylamine with ice cooling and stirring. The said acid chloride solution is added dropwise to the solution, while keeping the reaction mixture to pH 7.4 - 9.0 with triethylamine. After the dropwise addition, the reaction is continued for one hour, and the reaction mixture is adjusted to pH 2.0 with 1 N hydrochloric acid and then extracted three times with ethyl acetate. The organic layers are joined together, washed twice with 1 N hydrochloric acid and three times with a saturated aqueous sodium chloride solution, dried over anhydrous sodium sulfate and then concentrated under reduced pressure. Then, 10 ml of an aqueous 50% acetic acid solution is added to the residue, and the mixture is heated at 60°C with stirring for one hour and then concentrated under reduced pressure. The residue is washed twice with ether and dissolved in dimethylsulfoxide. The solution is charged onto 10 ml of Diaion HP-10, the resin is washed with water, and elution is carried out with a gradient from water to methanol - water = 5 / 1 (V/V) to obtain 105 mg of acyl compound.

NMR δ(DMSOd6 + $CD_3OD$): 8.0 - 7.8(2H, m), 7.40(1H, d, J = 7.5Hz), 6.80(1H, s), 6.30(1H, m), 6.51(1H, d, J = 5.2Hz), 5.37(2H, s), 4.95 - 4.65(1H, m), 2.28(6H, s), 2.6 - 1.2(4H, m)

IR $\nu_{max}^{KBr}$ ($cm^{-1}$): 3300, 2920, 1770, 1680, 1630, 1535

Example 15

Preparation of sodium salt of (6R, 7S)-7-[2-(2-aminothiazol-4-yl)-2-syn-(3,4-dihydroxybenzoylmethoxyimino)] acetamido-1-azabicyclo [4,2,0] oct-2-en-8-oxo-2-carboxylic acid:

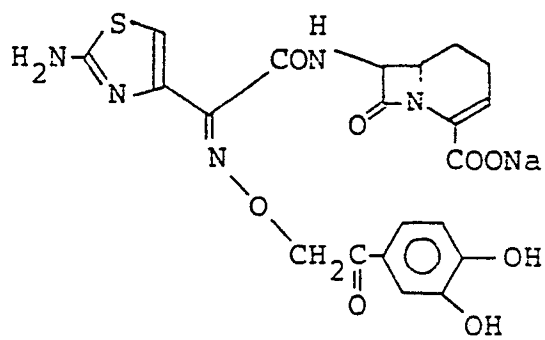

At first, 432 mg of (6R, 7S)-7-[2-(2-aminothiazol-4-yl)-2-syn-(3,4-diacetoxybenzoylmethoxyimino)] acetamido-1-azabicyclo [4,2,0] oct-2-en-8-oxo-2-carboxylic acid is suspended in 20 ml of water and 20 ml of methanol, and dissolved therein by making pH 7.0 with a saturated aqueous sodium bicarbonate solution with ice cooling and stirring. Then, the solution is adjusted to pH 10.5 with a solution prepared by two-fold dilution of concentrated aqueous ammonia, and stirred for 20 minutes after elevating the reaction temperature to room temperature. Then, acetic acid is added thereto to make pH 7.5, and then the mixture is concentrated under reduced pressure. The residue is dissolved in a saturated aqueous sodium bicarbonate solution, the solution is charged onto a column chromatograph with 50 ml of Diaion HP 10, and elution is carried out with a gradient from water to water - methanol = 1 / 1 (V/V). After concentration under reduced pressure, 174 mg of the desired compound is obtained.

NMR $\delta(D_2O)$: 7.61 - 7.37(2H, m), 6.98(1H, s), 6.85(1H, d, J = 8Hz), 6.16(1H, m), 5.48(1H, d, J = 4.9Hz), 5.38(2H, s), 4.02 - 3.82(1H, m), 2.3 - 1.6(4H, m)

IR $\nu_{max}^{KBr}$ ($cm^{-1}$): 3200, 1750(sh), 1745, 1680, 1660, 1590, 1535

Example 16

Preparation of 7-[2-(2-aminothiazol-4-yl)-2-syn-(3,4-diacetoxybenzoylmethoxyimino)] acetamido-3-acetoxymethyl-3-cephem-4-carboxylic acid:

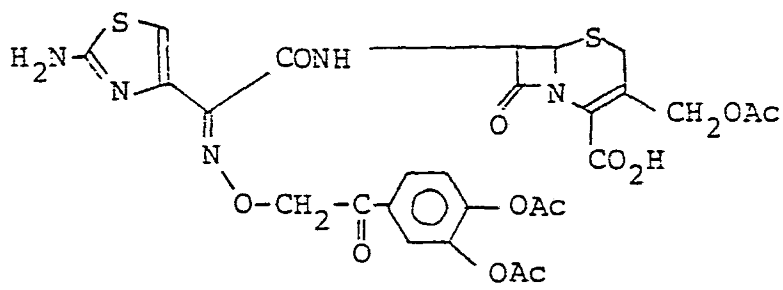

At first, 332 mg of 2-(tritylaminothiazol-4-yl)-2-syn-(3,4-diacetoxybenzoylmethoxyimino) acetic acid is dissolved in 5 ml of anhydrous tetrahydrofuran, and 69 μl of triethylamine is added thereto with cooling to -20°C and stirring. Then, 104 mg of phosphorus pentachloride is added thereto, and the mixture is stirred for 20 minutes to prepare an acid chloride solution.

Separately, 200 mg of 7-amino-3-acetoxymethyl-3-cephem-4-carboxylic acid is suspended in 5 ml of tetrahydrofuran and 5 ml of water, and dissolved therein with ice cooling and stirring by making pH 9.0 with triethylamine to make an amino acid solution. The said acid chloride solution is added dropwise to the amino acid solution with ice cooling and stirring, while keeping the reaction mixutre at pH 7.4 - 9.0 with triethylamine.

After the dropwise addition, reaction is continued for 30 minutes, and the reaction mixture is adjusted to pH 2.0 with 1 N hydrochloric acid, and extracted three times with ethyl acetate. The organic layers are joined together, washed three times with an aqueous sodium chloride solution, and dried over anhydrous sodium sulfate. After filtration, the filtrate is concentrated under reduced pressure, and 20 ml of an aqueous 50% acetic acid solution is added to the residue. The mixture is heated at 60°C with stirring for one hour, and then concentrated under reduced pressure

to obtain a crude acyl compound. The crude acyl compound is washed twice with ether, and then dissolved in DMSO. The solution is charged onto a column of 10 ml of Diaion HP 10, and elution is carried out with a gradient from water to water - methanol = 1 / 5 (V/V) to obtain 50 mg of desired compound.

NMR δ(DMSOd6 + $CD_3OD$): 8.0 - 7.8(2H, m), 7.44(1H, d, J = 8.3Hz), 6.84(1H, s), 5.37(1H, d, J = 4.9Hz), 5.41(2H, s), 5.18(1H, d, J = 4.9Hz), 5.05(1H, d, J = 12.9Hz), 4.72(1H, d, J = 12.9Hz), 3.68(1H, d, J = 18.3Hz), 3.43(1H, d, J = 18.3Hz), 2.31(6H, s), 2.04(3H, s)

IR $\nu_{max}^{KBr}$ ($cm^{-1}$): 3330, 2940, 1780, 1750(sh), 1690, 1630, 1600, 1540

Example 17

Preparation of 7-[2-(2-aminothiazolyl-4-yl)-2-syn-(3,4-diacetoxybenzoyloxyimino)] acetamido-3-(1-carboxymethyl-1H-tetrazol- 5 -ylthiomethyl)-3-cephem-4-carboxylic acid:

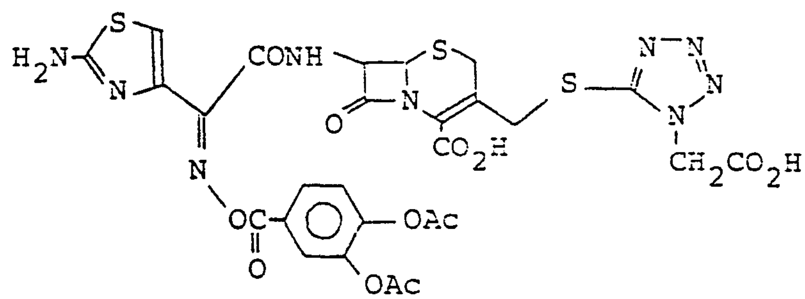

At first, 308 mg of 2-(2-tritylaminothiazol-4-yl)-2-syn-(3,4-diacetoxybenzoyloxyimino) acetic acid is dissolved in 3 ml of anhydrous tetrahydrofuran, and 69 mg of triethylamine is added thereto with cooling to -70°C. Successively, 105 mg of phosphorus pentachloride is added thereto, and then the mixture is stirred at 0°C for one hour to prepare an acid chloride solution.

Separately, 100 mg of 7-amino-3-(1-carboxymethyl-1H-tetrazol-5-ylthiomethyl)-3-cephem-4-carboxylic acid is

suspended in 5 ml of tetrahydrofuran and 5 ml of water, and dissolved therein with cooling to 0°C by making pH 9.0 with triethylamine. The said acid chloride solution is added dropwise to the solution while keeping the reaction mixture to pH 7.5 - 9.0 with triethylamine. After the dropwise addition, reaction is continued for 30 minutes, and then the reaction mixture is adjusted to pH 2.0 with 1 N hydrochloric acid and extracted three times with ethyl acetate. The organic layers are joined together, washed with a saturated aqueous sodium chloride solution, dried over sodium sulfate, and concentrated under reduced pressure. Then, 20 ml of 50% acetic acid is added to the residue, and the mixture is heated at 60°C with stirring for one hour, and then concentrated under reduced pressure. The residue is dissolved in a small amount of dimethylsulfoxide, and charged onto a column of 10 ml of Diaion HP 10. By elution with a gradient from water to water - methanol = 1 / 5 (V/V), 65 mg of acyl compound is obtained.

NMR δ(DMSOd6 + $CD_3OD$): 8.05 - 7.91(1H, m), 7.96(1H, s), 7.42 - 7.32(1H, m), 7.18(1H, s), 5.93(1H, d, J = 4.9Hz), 5.25(2H, s), 5.19(1H, d, J = 4.9Hz), 4.55(1H, d, J = 13.8Hz), 4.24(1H, d, J = 13.8Hz), 3.72(1H, s), 3.68(1H, s), 2.32(3H, s), 2.30(3H, s)

IR $\nu_{max}^{KBr}$ ($cm^{-1}$): 3200, 1780, 1755(sh), 1700, 1640, 1615

Example 18

Process for preparing 2-(2-tritylaminothiazol-4-yl)-2-syn-[1-(3,4-diacetoxybenzoyl) ethyl] oxyiminoacetic acid:

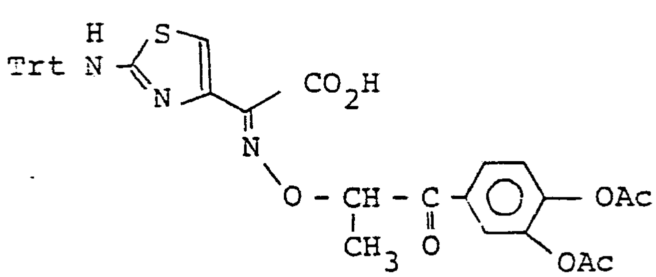

Preparation is made according to the following steps 1) and 2):

1) Preparation of diphenylmethyl 2-(2-tritylaminothiazol-4-yl)-2-syn-[1-(3,4-diacetoxybenzoyl) ethyl] oxyiminoacetate:

At first, 25 g of diphenylmethyl 2-(2-tritylaminothiazol-4-yl)-2-syn-hydroxyiminoacetate is dissolved in 5 ml of anhydrous dimethylsulfoxide, 9.01 g of anhydrous sodium carbonate is suspended therein, and the suspension is stirred.

Separately, 13.0 g of 1-bromoethyl-(3,4-dihidroxyphenyl) ketone is suspended in 500 mg of water, and 10.1 g of borax is added thereto with stirring. When the reaction solution becomes transparent, the solution is concentrated to dryness under reduced pressure, and then the residue is further dried over phosphorus pentoxide under reduced pressure to obtain a borate compound of catechol. The borate compound is added to the former suspension. The reaction mixture is stirred at room temperature for two hours, diluted with ethyl acetate, and washed with 1 N hydrochloric acid and twice with a saturated sodium chloride solution. The organic layer is dried over anhydrous sodium sulfate, and concentrated to one-third volume under reduced pressure. Then, 17.8 ml of anhydrous acetic acid and 24.1 ml of trithylamine are added to the concentrate,and the mixture is allowed to stand for one hour. The reaction mixture is washed with a saturated aqueous sodium bicarbonate solution, with 1 N hydrochloric acid and further three times with a saturated aqueous sodium chloride solution, then dried over anhydrous sodium sulfate and concentrated under reduced pressure. The residue is purified by silica gel column chromatography [cyclohexane / ethyl acetate = 3 / 1 (V/V)] to obtain 7.20 g of the desired compound.

NMR ($CDCl_3$) δ: 8.0 - 7.7(2H, m), 7.5 - 7.1(26H, m), 7.08(1H, s), 6.22(1H, s), 5.50(1H, q, J = 6Hz), 2.26(6H, s), 1.50(3H, s)

2) Preparation of 2-(2-tritylaminothiazol-4-yl)-2-syn-[1-(3,4-diacetoxybenzoyl) ethyl] oxyiminoacetic acid:

At first, 7.20 g of diphenylmethyl 2-[2-tritylaminothiazol-4-yl)-2-syn-[1-(3,4-diacetoxybenzoyl)-ethyl] oxyiminoacetate prepared in 1) is dissolved in 60 ml of anhydrous methylene chloride and 60 ml of nitromethane, and 5.5 g of aluminum chloride dissolved in 60 ml of nitromethane is added thereto dropwise with ice cooling and stirring. After the dropwise addition, the mixture is stirred for 5 minutes, diluted with ethyl acetate, and washed twice with 1 N hydrochloric acid and once with a saturated aqueous sodium chloride solution. The organic layer is dried over anhydrous sodium sulfate, and then concentrated under reduced pressure. Then, 60 ml of n-hexane and 60 ml of ether are added to the residue, and the mixture is stirred for pulverization, and filtered to obtain 3.77 g of the desired carboxylic acid.

NMR ($CDCl_3$) δ: 8.1 - 7.7(2H, m), 7.5 - 7.2(16H, m), 6.68(1H, s), 5.62(1H, q, J = 6Hz), 2.27(6H, s), 1.57(3H, d, J = 6Hz)

Example 19

Preparation of (6R, 7S)-7-{2-(2-aminothiazol-4-yl)-2-syn-[1-(3,4-diacetoxybenzoyl) ethyl] oxyimino} acetamido-1-azabicyclo [4,2,0] oct-2-en-8-oxo-2-carboxylic acid:

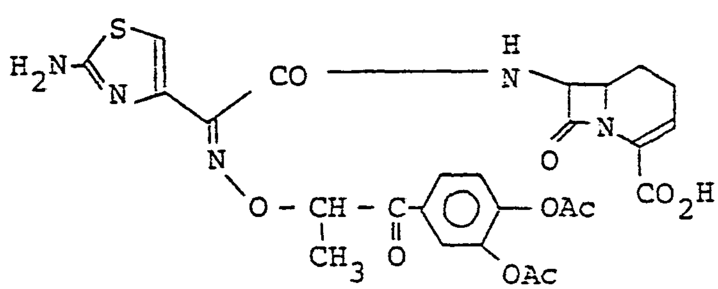

At first, 3.77 g of 2-(2-tritylaminothiazol-4-yl)-2-syn-[1-(3,4-diacetoxybenzoyl) ethyl] oxyiminoacetic acid synthesized in Example 18 is dissolved in 30 ml of anhydrous tetrahydrofuran, and 0.77 ml of triethylamine is added

thereto with ice cooling. Then, 1.16 g of phosphorus pentachloride is added to the mixture with stirring, and the mixture is further stirred for 30 minutes to prepare an acid chloride solution.

Separately, 2.00 g of (6R, 7S)-7-amino-1-azabicycle [4,2,0] oct-2-en-8-oxo-2-carboxylic acid is suspended in 20 ml of tetrahydrofuran and 20 ml of water, and the suspension is adjusted to pH 9.0 with triethylamine with ice cooling and stirring to dissolve the carboxylic acid.

The said acid chloride solution is added dropwise to the solution. At the same time, triethylamine is added thereto to keep the pH of the reaction mixture at 7.4 - 9.0. After the dropwise addition, the reaction is continued for one hour, and the reaction mixture is adjusted to pH 2.0 with 1 N hydrochloric acid. The reaction mixture is then extracted three times with ethyl acetate, and the organic layers are joined together, washed three times with a saturated aqueous sodium chloride solution, then dried over anhydrous sodium sulfate and concentrated under reduced pressure. Then, 100 ml of acetic acid, 30 ml of methanol and 100 ml of water are added to the residue, and the mixture is heated at 50°C for one hour with stirring and then concentrated under reduced pressure. The residue is washed twice with ether to obtain a crude acyl compound.

The crude acyl compound is dissolved in dimethylsulfoxide, and the solution is charged onto a column of 200 ml of Diaion HP 10, and fractions, which are eluted with water - methanol = 1 / 3 (V/V) are collected, whereby 570 mg of the desired compound is obtained.

NMR ($DMSOd_6$ + $CO_3OD$) δ: 8.2 - 7.7(2H, m), 7.4(1H, d, J = 8.1Hz), 6.80(1H, s), 6.33(1H, m), 6.7 - 6.4(2H, m), 2.30(6H, s), 1.50(3H, d, J = 6Hz)

IR $\nu_{max}^{KBr}$ ($cm^{-1}$): 3350, 2950, 1780, 1690, 1645

Example 20

Preparation of 2-(2-tritylaminothiazol-4-yl)-2-syn-[1-(3,4-diacetoxybenzoyl)-1-methylethyl] oxyiminoacetic acid:

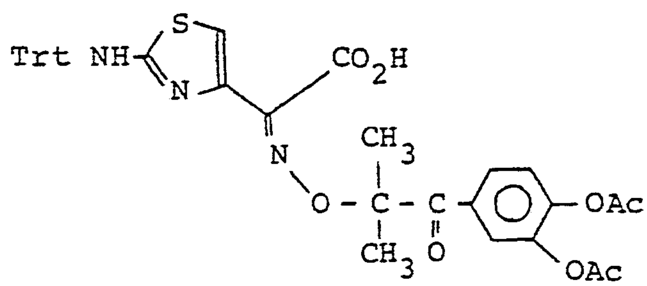

Preparation is made according to the following steps 1) and 2):

1) Synthesis of diphenylmethyl 2-(2-tritylaminothiazol-4-yl)-2-syn-[1-(3,4-dihydroxybenzoyl)-1-methylethyl] oxyiminoacetate:

At first, 3.42 g (6.0 mmoles) of diphenylmethyl 2-(2 - tritylaminothiazol-4-yl)-2-syn-hydroxyiminoacetate is dissolved in 12 ml of anhydrous dimethylsulfoxide, and then 2.55 g of anhydrous potassium carbonate is suspended therein.

Separately, 2.12 g of 1-bromo-1-methylethyl 3,4-dihydroxyphenyl ketone [J. Med. Chem. 7 178 (1964)] is suspended in 10 ml of water, and then 1.56 g of borax is added thereto with stirring. When the reaction mixture turns into a homogeneous solution, the mixture is concentrated under reduced pressure, and then concentrated to dryness, while adding toluene several times thereto to obtain a borate compound.

The borate compound is added to the said suspension, and the mixture is stirred at room temperature for 16 hours, diluted with 100 ml of ethyl acetate, washed twice with 1.0 N hydrochloric acid and three times with a saturated aqueous sodium chloride solution. The organic layer is dried over sodium sulfate and concentrated under reduced

pressure. Then, 4.7 g of the residue is purified by silica gel chromatography [160 g of silica gel, 1.2 ℓ of developing solution (hexane / ethyl acetate = 4 / 1), 1.2 ℓ of the solution (hexane / ethyl acetate = 3 / 1), 1.5 ℓ of the solution (hexane / ethyl acetate = 2 / 1, one fraction = 17 - 18 μl]. Fractions Nos. 106 - 170 are joined together, and concentrated under reduced pressure to obtain 1.8 g of the desired compound (yield: 40%).

IR $\nu_{max}^{KBr}$ ($cm^{-1}$): 1759, 1750, 1740, 1600, 1540, 700

NMR ($CDCl_3$) δ: 6.18(1H, s), 1.40(6H, s)

2) Synthesis of diphenylmethyl 2-(2-tritylaminothiazol-4-yl)-2-syn-[1-(3,4-diacetoxybenzoyl)-1-methylethyl] oxyiminoacetate:

At first, 903 mg of diphenylmethyl 2-(2-tritylaminothiazol-4-yl)-2-syn-[1-(3,4-dihydroxybenzoyl)-1-methylethyl] oxyiminoacetate synthesized in 1) is dissolved in 5 ml of pyridine, and then 1 ml of anhydrous acetic acid is added thereto. The mixture is stirred at room temperature for 6 hours, and then 50 ml of ethyl acetate is added thereto. The mixture is washed with 1.0 N HCl and with a saturated aqueous sodium chloride solution, dried over sodium sulfate and concentrated under reduced pressure to obtain 1.1 g of the residue. Then, the residue is triturated in hexane-ether, whereby 970 mg of the desired compound is obtained (yield: 96%).

IR $\nu_{max}^{KBr}$ ($cm^{-1}$): 1784, 1780, 1758, 1750, 1695, 1540

NMR ($CDCl_3$) δ: 7.9(2H, m), 7.0(1H, d, J = 7.5Hz), 6.40(1H, s), 2.26(3H, s), 2.20(3H, s), 1.48(6H, s)

3) Synthesis of 2-(2-tritylaminothiazol-4-yl)-2-syn-[1-(3,4-diacetoxybenzoyl)-1-methylethyl] oxyiminoacetic acid:

At first, 950 mg of diphenylmethyl 2-(2-tritylaminothiazol-4-yl)-2-syn-[1-(3,4-diacetoxybenzoyl)-1-methylethyl] oxyiminoacetate is dissolved in 6 ml of methylene

chloride, 6 ml of nitromethane, and 0.5 ml of anisole, and 451 mg of aluminum chloride dissolved in 4 ml of nitromethane is added thereto dropwise with ice cooling and stirring. After the dropwise addition, the mixture is stirred for 5 minutes, and 60 ml of ethyl acetate is added thereto. The mixture is then washed with 1 N HCl and with a saturated aqueous sodium chloride solution, dried over sodium sulfate, and concentrated under reduced pressure to obtain 1.02 g of the residue. The residue is triturated in 10 ml of hexane, whereby 785 mg of the desired compound is obtained (yield: 100%).

IR $\nu_{max}^{KBr}$ $(cm^{-1})$: 1782, 1775, 1515, 1205, 700

NMR $(CDCl_3)\delta$: 8.1(2H, m), 6.22(1H, s), 2.23(3H, s), 2.13(3H, s), 1.21(6H, s)

Example 21

Preparation of a triethylamine salt of [6R, 7S]-7-[2-(2-aminothiazol-4-yl)-2-syn-[1-(3,4-diacetoxybenzoyl)-1-methylethyl] oxyiminoacetamido]-1-azabicyclo [4,2,0] oct-2-en-8-oxo-2-carboxylic acid:

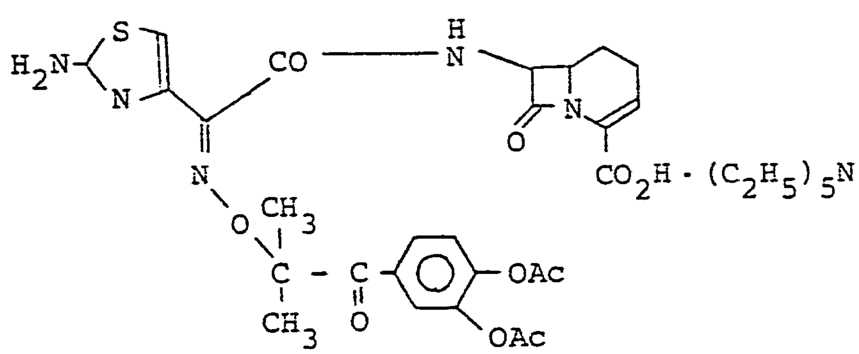

At first, 0.59 g of 2-(2-tritylaminothiazol-4-yl)-2-syn-[1-(3,4-diacetoxybenzoyl)-1-methylethyl] oxyiminoacetic acid synthesized in Example 20 is dissolved in 4 ml of anhydrous tetrahydrofuran, and 239 µl of triethylamine is added thereto with ice cooling and stirring. Then, 198 µl of phosphorus oxychloride is added thereto. The mixture is stirred with ice cooling for 30 minutes, and at room temperature

0075805

for 30 minutes to prepare an acid chloride—THF solution.

Separately, 80 mg of [6R, 7S]-7-amino-1-azabicyclo-[4,2,0]-oct-2-en-8-oxo-2-carboxylic acid is dissolved in 2 ml of water and 2 ml of THF, and the solution is adjusted to pH 7.5 with triethylamine with ice cooling. Then, the said acid chloride solution is added thereto dropwise with ice cooling while keeping the solution at pH 7.0 - 7.5 with triethylamine. After stirring at 0°C for 40 minutes, the mixture is adjusted to pH 2.0 with 1 N HCl. After saturation with sodium chloride, the mixture is extracted with ethyl acetate, and the organic layer is washed with an aqueous sodium chloride solution, dried over sodium sulfate and concentrated under reduced pressure to obtain 620 mg of crude acyl compound.

The crude acyl compound is dissolved in 3 ml of methanol / water (= 10 / 1), and the solution is adjusted to pH 1.5 with 1.0 N HCl and stirred at 50°C for 30 minutes. The solution is adjusted to pH 7.0 with triethylamine, and concentrated under reduced pressure. Then, the residue is titulated in ether, and purified with Diaion HP-10.

HP-10 60 cc
Water 60 ml
Water-methanol solution : 60 ml (2/1); 60 ml (1/1); 60 ml (1/2); 60 ml (1/3); 60 ml (1/5)
One fraction: 7 - 8 ml

Fractions Nos. 42 - 48 are joined together, and concentrated under reduced pressure to obtain 108 mg of the desired compound (yield: 34%).

IR $\nu_{max}^{KBr}$ ($cm^{-1}$): 1792, 1785, 1780, 1690, 1518, 1205, 700

NMR ($CD_3OD$) δ: 8.06(2H, m), 7.30(1H, d, J = 8Hz), 6.77(1H, s), 6.07(1H, m), 5.48(1H, d, J = 4.5Hz), 3.9(1H, m), 3.17(6H, q, J = 7.5Hz), 2.28(6H, s), 1.67(3H, s), 1.63(3H, s), 1.28(9H, t, J = 7.5Hz)

0075805

Example 22

Preparation of (6R, 7R)-7-[2-(2-aminothiazol-4-yl)-2-syn-[1-(3,4-diacetoxybenzoyl)-1-methylethyl] oxyimino-acetamido]-3-(1-carboxymethyl-5-tetrazolyl) thiomethyl-$\Delta^3$-cephem-4-carboxylic acid:

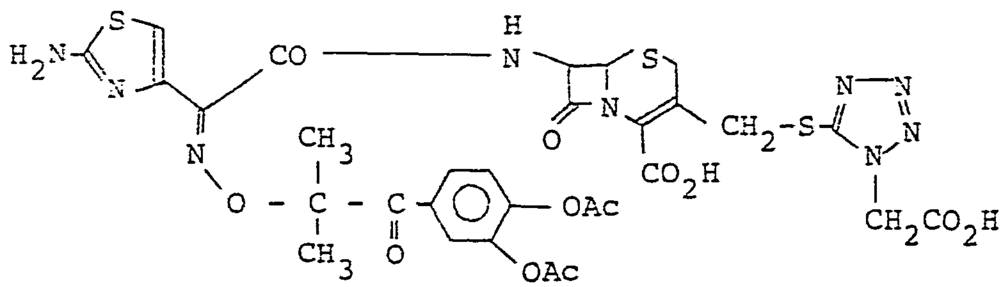

Synthesis is made according to the procedure of Example 20.

At first, 1 g of 2-(2-tritylaminothiazol-4-yl)-2 syn-[1-(3,4-diacetoxybenzoyl)-1-methylethyl] oxyiminoacetic acid is dissolved in 10 ml of anhydrous tetrahydrofuran, and 202 µl of triethylamine is added thereto. The mixture is cooled to -15°C with stirring. Then, 335 µl of phosphorus oxychloride is added thereto, and the mixture is stirred for 60 minutes to prepare an acid chloride solution.

Separately, 538 mg of (6R, 7R)-7-amino-3-(1-carboxymethyl-5-tetrazolyl) thiomethyl-$\Delta^3$-cephem-4-carboxylic acid is suspended in 20 ml of tetrahydrofuran and 20 ml of water, and the mixture is adjusted to pH 7.7 with triethylamine with ice cooling and stirring to dissolve the carboxylic acid. The said acid chloride solution is added dropwise to the solution, and at the same time triethylamine is added thereto dropwise to keep the reaction mixture at pH 7.0 - 8.5. After the dropwise addition, reaction is continued for one hour. Then, the reaction mixture is adjusted to pH 2.5 with 1 N hydrochloric acid, and extracted three times with ethyl acetate. The organic layers are joined together, washed three times with a saturated aqueous sodium chloride solution, dried over anhydrous sodium sulfate and concentrated under reduced pressure. Then, 80 ml of methanol / water (10 / 1)

is added to the residue, and the mixture is adjusted to pH 1.5 with 1 N hydrochloric acid and heated at 50°C with stirring for one hour. Then, the mixture is adjusted to pH 7.0 with triethylamine, and concentrated under reduced pressure. Then, 10 ml of water and 2 ml of dimethylsulfoxide are added to the residue, and the mixture is adjusted to pH 2.0 with 1 N hydrochloric acid and concentrated under reduced pressure. The residue is dissolved in dimethylsulfoxide, and the solution is charged onto 500 ml of Diaion HP-10, the resin is washed with water, and elution is carried out with methanol / water while changing the ratio from 1/5 to 2/1 for each 1 ℓ volume, whereby 382 mg of the desired compound is obtained.

NMR ($DMSOd_6 + CD_3OD$) δ: 8.2 - 8.0(2H, m), 7.30(1H, d, J = 8.7Hz), 6.8(1H, s), 5.83(1H, d, J = 5.2Hz), 5.22(2H, s), 5.16(1H, d, J = 5.2Hz), 3.35 - 4.80 (4H, m), 2.27(6H, s), 1.60(6H, s)

IR $\nu_{max}^{KBr}$ ($cm^{-1}$): 3400, 1775, 1675, 1620, 1525

Example 23

Preparation of (6R, 7R)-7-[2-(2-aminothiazol-4-yl)-2-syn-[1-(3,4-diacetoxybenzoyl)-1-methylethyl] oxiyminoacetamido]-3-(1-pyridiniummethyl)-3-cephem-4-carboxylate:

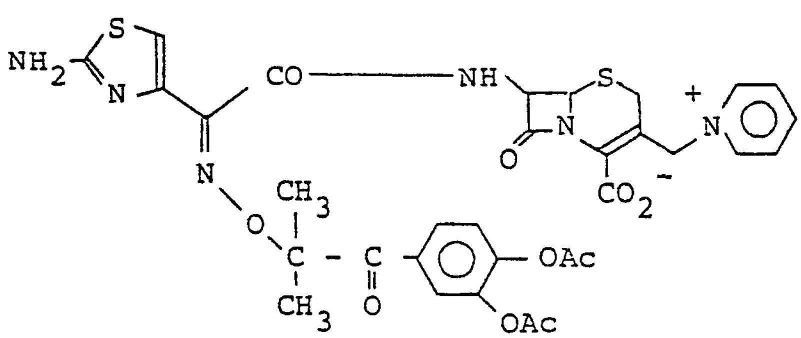

At first, 2.44 g of 2-(2-tritylaminothiazol-4-yl)-2-syn-[1-(3,4-diacetoxybenzoyl)-1-methylethyl] oxyiminoacetic acid synthesized according to the procedure of Example 20 is dissolved in 35 ml of anhydrous methylene chloride, and the

solution is cooled to -20°C with stirring. Then, 1.77 g of phosphorus pentachloride is added thereto, and the mixture is stirred at the same temperature for 50 minutes. Then, 105 ml of diisopropyl ether is added to the resulting solution, and the mixture is stirred with ice cooling for 40 minutes. The deposited precipitate is separated by filtration.

Separately, a stirred suspension of 1.32 g of (6R, 7R)-7-amino-3-(1-pyridiniummethyl)-3-cephem-4-carboxylic acid dihydrochloride in a mixture of 18 ml of N,N-dimethylacetamide and 18 ml of acetonitrile, which mixture is containing 2.52 ml of triethylamine, is cooled to -10°C. A solution of the said precipitate in 35 ml of anhydrous methylene chloride is added dropwise to the cooled suspension over 10 minutes.

The mixture is stirred at -10°C for 30 minutes and at room temperature for 40 minutes. Then, 1 ml of methanol is added to the reaction mixture, and the resulting mixture is concentrated under reduced pressure. The resulting N,N-dimethylacetamide solution is added dropwise to 180 ml of water with stirring. After stirring with ice cooling for one hour, the resulting precipitate is separated by filtration and dissolved in 300 ml of chloroform. The solution is washed with a saturated aqueous sodium chloride solution, dried over anhydrous sodium sulfate, and concentrated under reduced pressure. Then, 50 ml of 50% acetic acid - water (V/V) is added to the residue, and the mixture is stirred at 50°C for one hour. The deposited insoluble matters are filtered off, and the filtrate is concentrated under reduced pressure. The residue is dissolved into dimethylsulfoxide, and the solution is charged onto 500 ml of Diaion HP-10, and elution is carried out in the order of: 1,000 ml of water, 1,000 ml of water / methanol (4 / 1), 900 ml of water / methanol (2 / 1), 2,500 ml of water / methanol (1 / 1), and 2,000 ml of water / methanol (1 / 2). The fractions containing the desired compound are concentrated under reduced pressure, whereby 630 mg of the desired compound is obtained (yield: 24%).

IR $\nu_{max}^{KBr}$ ($cm^{-1}$): 3400, 1780, 1680, 1630, 1540, 1210

NMR ($DMSOd_6$ + $CD_3OD$) $\delta$ (ppm): 9.5 - 9.0 (2H, m), 8.4 - 8.7 (1H, m), 8.3 - 7.8 (4H, m), 7.4 - 7.2 (1H, m), 6.75 (0.4H, s), 6.67 (0.6H, s), 5.79 (1H, d, J = 4.9 Hz), 5.7 - 5.2 (2H, m), 5.15 (1H, d, J = 4.9 Hz), 3.0 - 3.8 (2H, m), 2.29 (6H, s), 1.58 (2.4H, s), 1.54 (3.6H, s)

Example 24

Preparation of (6R, 7R)-7-{2-(2-aminothiazol-4-yl)-2-syn-[1-(3,4-diacetoxybenzoyl)-1-methylethyl] oxyiminoacetamido}-3-acetoxymethyl-3-cephem-4-carboxylic acid:

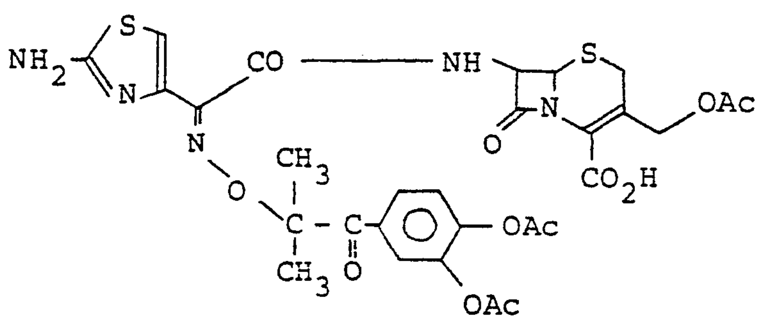

At first, 1.73 g of 2-(2-tritylaminothiazol-4-yl)-2-syn-[1-(3,4-diacetoxybenzoyl)-1-methylethyl] oxyiminoacetic acid synthesized according to the process of Example 20 is dissolved in 25 ml of anhydrous tetrahydrofuran, and the solution is cooled to -10°C with stirring. After 0.35 ml triethylamine is added thereto, 521 mg of phosphorus pentachloride is added thereto, and the mixture is stirred at the same temperature for 30 minutes to prepare an acid chloride solution.

On the other hand, 25 ml of water and 25 ml of tetrahydrofuran are added to 730 mg of 7-aminocephalosporanic acid (purity: 92.9%), and the mixture is adjusted to pH 9.5 with triethylamine. The said acid chloride solution is added dropwise to the mixture over 20 minutes, while adjusting the mixture to pH 8 - 8.5 with triethylamine.

After stirring for 40 minutes with ice cooling, the reaction mixture is adjusted to pH 2.5 with 6 N hydrochloric acid, and then extracted twice with ethyl acetate. After washing three times with a saturated aqueous sodium chloride solution, the extract is dried over anhydrous sodium sulfate, and concentrated under reduced pressure. Then, 20 ml of acetic acid is added to the residue, and the resulting solution is heated to 55°C. Then, 20 ml of water is added thereto over 10 minutes, and the mixture is stirred at the same temperature for additional 30 minutes.

The deposited insoluble matters are separated by filtration, and the filtrate is concentrated under reduced pressure. After water is added to the residue, the mixture is extracted twice with ethyl acetate. The extract is washed twice with a saturated aqueous sodium chloride solution, dried over anhydrous sodium sulfate, and concentrated under reduced pressure. The residue is dissolved in dimethylsulfoxide, and the solution is charged onto 150 ml of Diaion HP-10, and elution is carried out with 300 ml of water, 300 ml of water : methanol = 1 : 2, and 600 ml of water : methanol = 1 : 8 in this order, and fractions containing the desired compound are collected, and distilled under reduced pressure. The thus obtained aqueous solution is extracted twice with ethyl acetate.

After drying over sodium sulfate, the extract is concentrated under reduced pressure to obtain 790 mg of the desired compound (yield: 45%).

NMR (δ ppm in $CDCl_3$-$CD_3OD$): 1.67(6H, s), 2.07(3H, s), 2.23(3H, s), 2.25(3H, s), 3.36, 3.66(2H, ABq, J = 19 Hz), 4.89, 5,13(2H, ABq, J = 14 Hz), 5.12 (1H, d, J = 5 Hz), 5.93(1H, d, J = 5 Hz), 6.67(1H, s), 7.22(1H, d, J = 9 Hz), 7.9 - 8.1(2H, m)

IR $\nu_{max}^{KBr}$ ($cm^{-1}$): 3300, 1780, 1695, 1685(sh), 1630, 1540

Example 25

Preparation of sodium (6R, 7R)-7-{2-(2-aminothiazol-4-yl)-2-syn-[1-(3,4-dihydroxybenzoly)-1-methylethyl] oxyiminoacetamido}-3-acetoxymethyl-3-cephem-4-carboxylate

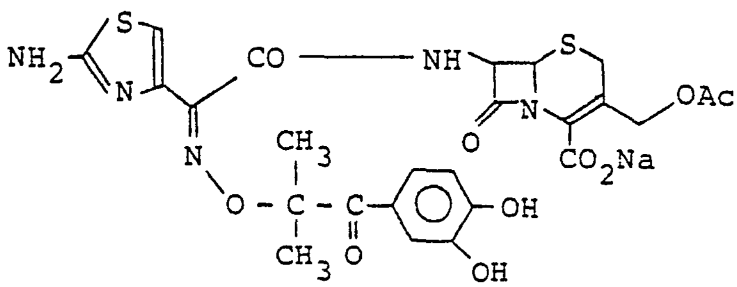

At first, 550 ml of methanol is added to 14.08 g of (6R, 7R)-7-{2-(2-aminothiazol-4-yl)-2-syn-[1-(3,4-diacetoxybenzoyl)-1-methylethyl] oxyiminoacetamido}-3-acetoxymethyl-3-cephem-4-carboxylic acid synthesized according to the process of Example 24. The resulting solution is ice-cooled, and 0.79 ml of a concentrated aqueous ammonia (28 %) is added thereto. After stirring at the same temperature for 1.5 hours, concentrated hydrochloric acid is added thereto to adjust pH to about 7. Then, the reaction mixture is concentrated under reduced pressure, an aqueous sodium bicarbonate solution is added to the residue, and the resulting solution is charged onto 1 ℓ of Diaion HP-10. Elution is carried out with 2 ℓ of water, 3 ℓ of water : methanol = 9 : 1, and 3.5 ℓ of water : methanol = 1 : 2 in this order, and fractions containing the desired compound are collected and concentrated under reduced pressure to obtain 9.10 g of the desired compound (yield: 71%).

NMR ($\delta$ ppm in $D_2O$): 1.62(6H, s), 2.07(3H, s), 3.31, 3.62(2H, ABq, J = 17 Hz), 4.68, 4.89(2H, ABq, J = 12 Hz), 5.17(1H, d, J = 5 Hz), 5.82(1H, d, J = 5 Hz), 6.82(1H, s), 6.87(1H, d, J = 9 Hz), 7.5 - 7.8(2H, m)

IR $\nu_{max}^{KBr}$ ($cm^{-1}$): 3300, 1780, 1670, 1600, 1530

Example 26

Preparation of sodium (6R, 7R)-7-{2-(2-amino-thiazol-4-yl)-2-syn-[1-(3,4-dihydroxybenzoyl)-1-methylethyl] oxyiminoacetamido}-3-(4-sulfoethylpyridinium)-methyl-3-cephem-4-carboxylate:

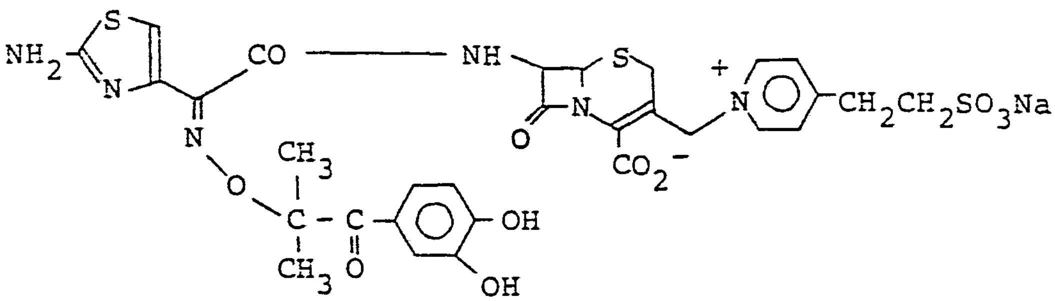

At first, 2.5 ml of water is added to 321 mg of (6R, 7R)-7-{2-(2-aminothiazol-4-yl)-2-syn-[1-(3,4-dihydroxybenzoyl-1-methylethyl] oxyiminoacetamido}-3-acetoxymethyl-3-cephem-4-carboxylate and 187 mg of 2-(4-pyridyl) ethanesulfonic acid, and then 84 mg of sodium bicarbonate is gradually added thereto. Then, 2.0 g of the sodium iodide is added to the resulting solution, and the mixture is stirred at 75°C for 2.5 hours. Then, the mixture is cooled to room temperature, and charged onto 50 ml of Diaion HP-10. Elution is carried out with 200 ml of water, 100 ml of water : methanol = 9 : 1, 100 ml of water : methanol = 6 : 1, and 100 ml of water : methanol = 3 : 1 in this order, and fractions containing the desired compound are collected and concentrated under reduced pressure to obtain 90 mg of crude product. The crude product is dissolved in water, and the solution is again charged onto 10 ml of Diaion HP-10. Elution is carried out with 40 ml of water, and 40 ml of water : methanol = 5 : 1 in this order, and fractions containing the desired compound are concentrated under reduced pressure to obtain 44 mg of the desired compound (yield: 12%).

NMR (δ ppm in $D_2O$): 1.54(3H, s), 1.60(3H, s), 3.24 (4H, s), 3.0 - 3.7(2H, m), 5.22(1H, d, J = 5 Hz), 5.0 - 5.7(2H, m), 5.84(1H, d, J = 5 Hz), 6.77(1H, s), 6.6 - 6.8(1H, m), 7.4 - 7.8(2H, m), 7.9(2H, d), 8.7(2H, d)

IR $\nu_{max}^{KBr}$ ($cm^{-1}$): 3400, 1780, 1670, 1610, 1540

Reference Example 1

Preparation of (6R, 7S)-7-[2-(2-aminothiazol-4-yl)-2-syn-benzoyloxyiminoacetamido]-1-azabicyclo [4,2,0] oct-2-en-8-oxo-2-carboxylic acid:

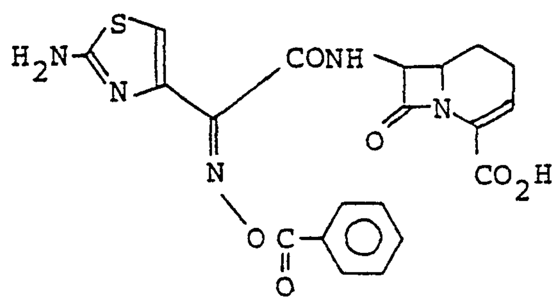

At first, 490 mg of 2-(2-tritylaminothiazol-4-yl)-2-syn-benzoyloxyiminoacetic acid is suspended in 10 ml of anhydrous methylene chloride, and 126 μl of triethylamine and 191 mg of phosphorus pentachloride are added thereto with cooling to -30°C and stirring. The mixture as such is brought back to room temperature over 30 minutes, and then concentrated under reduced pressure. The residue is dissolved in 10 ml of tetrahydrofuran to prepare an acid chloride solution.

Separately, 170 mg of (6R, 7S)-7-amino-1-azabicyclo [4,2,0] oct-2-en-8-oxo-2-carboxylic acid is suspended in 15 ml of tetrahydrofuran and 15 ml of water, and the suspension is adjusted to pH 8.5 with triethylamine with ice cooling and stirring to dissolve the carboxylic acid. The said acid chloride solution is added thereto dropwise, while maintaining the reaction mixture to pH 7.5 to 8.5 with

triethylamine. Then, 30 minutes after the dropwise addition, the mixture is adjusted to pH 2.0 with 1 N hydrochloric acid, and ethyl acetate is added thereto. The resulting two layers are separated, and the aqueous layer is extracted twice with ethyl acetate. The organic layers are joined together, washed twice with a saturated aqueous sodium chloride solution, dried over sodium sulfate, and concentrated under reduced pressure. Then, 50 ml of 50% acetic acid is added to the residue, and the mixture is stirred with heating at 60°C for one hour, and concentrated under reduced pressure. Then, 10 ml of ethyl acetate and 20 ml of ether are added to the residue, and after stirring for 30 minutes, the solvents are removed by decantation, and the residue is dried. The resulting powder is disssolved in dimethylsulfoxide, charged onto 10 ml of Diaion HP-10, and subjected to elution with a gradient from water to 80% methanol - 20% water, whereby 193 mg of acyl compound is obtained.

NMR ($CD_3OD$) δ: 8.2 - 7.8(2H, m), 7.7 - 7.3(3H, m), 7.20(1H, s), 6.46(1H, m), 5.67(1H, d, J = 5Hz), 4.1 - 3.75(1H, m), 2.4 - 1.3 (4H, m)

IR $\nu_{max}^{cm^{-1}}$ (KBr): 3300, 1760, 1680, 1660, 1630, 1540

Reference Example 2

Preparation of 3,4-bisdihydropyranyloxybenzyl chloride:

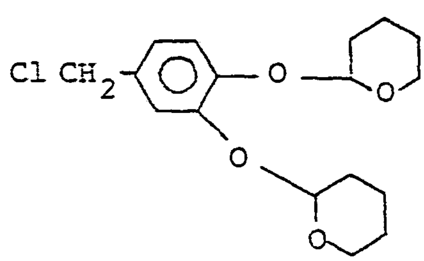

It is prepared according to the following steps 1) and 2).

1) Preparation of 3,4-bisdihydropyranyloxybenzyl alcohol:

At first, 7.0 g of 3,4-dihydroxybenzoic acid is dissolved in 10 ml of ethanol and 80 ml of methylene chloride, and a catalytic amount of p-toluenesulfonic acid is added thereto. In a Soxhlet extractor filled with molecular sieve 3A (made by Wako Junyaku Co.), the mixture is subjected to reaction with heating and refluxing for 3 hours, and is concentrated as such to obtain 6.9 g of crude ethyl ester compound. The crude ethyl ester compound as such is dissolved in 70 ml of anhydrous ether, and 9.2 ml of 2,3-dihydropyran and a catalytic amount of p-toluenesulfonic acid are added thereto. The mixture is subjected to reaction at room temperature for 3 hours, and then after further addition of 2 ml of 2,3-dihydropyran thereto, the mixture is subjected to reaction overnight. Then, 100 ml of anhydrous ethylene glycol dimethyl ether is added thereto, and 3.42 g of lithium aluminum hydride is added thereto by portions. The mixture is stirred for one hour. Then, water and ethyl acetate are added thereto, and the resulting two layers are separated. The aqueous layer is further extracted three times with ethyl acetate, and the organic layers are joined together, washed with water, dried over sodium sulfate and concentrated under reduced pressure. The residue is purified by silica gel column chromatography (Wako gel C-200 300 g; n-hexane-ethyl acetate 3 : 2 V/V) to obtain 445 mg of desired 3,4-dihydropyranyloxybenzyl alcohol.

2) Preparation of 3,4-bisdihydropyranyloxybenzyl chloride:

At first, 2.84 g of 3,4-bisdihydropyranyloxybenzyl alcohol prepared in the foregoing Reference Example 2 - 1) is dissolved in 60 ml of anhydrous ether and 5 ml of pyridine, and 1.7 ml of thionyl chloride dissolved in 20 ml of anhydrous ether is added thereto dropwise with ice cooling and stirring. The mixture is subjected to reaction for one hour after the dropwise addition, washed once with a saturated aqueous

sodium chloride solution and twice with water, dried with sodium sulfate, and concentrated to obtain desired 3,4-dihydroxybenzyl chloride.

Reference Example 3

Antibacterial activites by dilution method on Mueller-Hinton agar are shown in the following Table.

| Strain | Compound of Ex. 4 | Compound of Ex. 7 | Compound of Ex. 8 | Compound of Ex.10 | Compound of Ex.11 | Compound of Ex.12 | Compound of Ex.14 | Compound of Ex.19 |
|---|---|---|---|---|---|---|---|---|
| Staphilococcus aureus Smith | 6.25 | 50 | 6.25 | 12.5 | 3.13 | 6.25 | 3.13 | 12.5 |
| E. coli NIH JC-2 | 0.05 | 0.78 | 0.2 | 0.2 | 0.1 | 0.05 | 0.05 | 0.2 *1 |
| Klebsiella pneumoniae Y-60 | ≤ 0.01 | 0.1 | 0.1 | 0.1 | 0.02 | ≤ 0.01 | 0.02 | 0.39 |
| Serratia marcescens T-26 | 0.02 | 0.2 | 0.1 | 0.78 | 0.02 | 0.02 | 0.39 | 0.78 *2 |
| Proteus mirabilis 1287 | 0.1 | 0.78 | 0.1 | 0.1 | 0.1 | 0.05 | 0.05 | - |
| Proteus vulgaris 6897 | ≤ 0.01 | 0.1 | ≤ 0.01 | 0.1 | 0.02 | 0.02 | ≤ 0.01 | 0.1 |
| Proteus morganii 4298 | 0.1 | 1.56 | 0.2 | 0.39 | 0.2 | 0.02 | 0.1 | - |
| Proteus rettgeri 4289 | ≤ 0.01 | ≤ 0.01 | ≤ 0.01 | ≤ 0.01 | ≤ 0.01 | ≤ 0.01 | ≤ 0.01 | - |
| Enterobacter cloacae F-1510 | 0.05 | 0.2 | 0.39 | 0.2 | 0.1 | ≤ 0.01 | ≤ 0.01 | 3.13 *3 |
| Enterobacter aerogenes F-1949 | ≤ 0.01 | 0.1 | 0.05 | 0.1 | 0.02 | ≤ 0.01 | ≤ 0.01 | - |
| Citrobacter freundii F 1526 | ≤ 0.01 | 0.05 | 0.02 | 0.1 | ≤ 0.01 | ≤ 0.01 | ≤ 0.01 | - |
| Pseudomonas aerginosa 145 | 0.05 | 0.78 | 0.2 | 3.13 | 0.05 | 0.1 | 0.05 | 0.39 |
| Pseudomonoas sepatia F 2251 | 0.02 | 0.2 | 0.1 | 0.2 | 0.05 | 0.02 | ≤ 0.01 | - |
| Acinetobacter F 2575 | 12.5 | > 100 | > 100 | 100 | 12.5 | 12.5 | 12.5 | - |

*1 E. coli Juhl, *2 Serratia marcescens T-55, *3 Enterobacter cloacae F 1870

0075805

Reference Example 4

Preparation of (4S, 6R, 7S)-7-amino-4-methoxy-1-azabicyclo [4,2,0] oct-2-en-8-oxo-2-carboxylic acid:

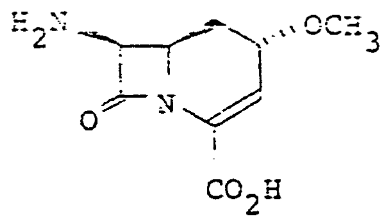

4-1) Preparation of tertiary butyl ester of (±)-cis-7β-phthalimido-4α-methoxy-1-azabicyclo [4,2,0] oct-2-en-8-oxo-2-carboxylic acid:

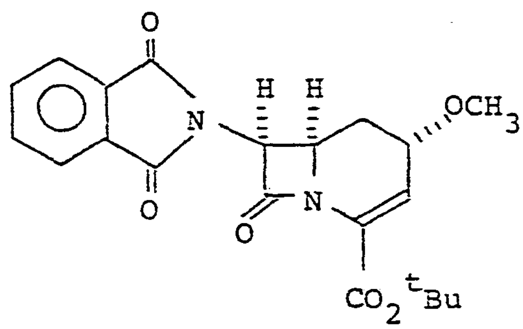

At first, 62 ml of anhydrous carbon tetrachloride, 0.59 g of N-bromosuccinimide and 0.19 g of azobisisobutyronitrile are added to 1.23 g of tertiarybutyl ester of (±)-cis-7β-phthalimido-1-azabicyclo [4,2,0] oct-en-8-oxo-2-carboxylic acid prepared in the same manner as in Reference Example 5, and the mixture is refluxed with heating and stirring for 40 minutes. Then, 10 ml of anhydrous methanol is added to the reaction mixture after the temperature is lowered to 70°C, and the resulting mixture is refluxed with heating for 20 minutes. The reaction mixture is concentrated under reduced pressure, and 5 ml of ethyl acetate, 10 ml of ether and 15 ml of n-hexane are added to the residue. The deposited crystals are separated by filtration to obtain 0.87 g of the desired compound (65.5%).

Physical properties of the compound are as follows:

IR $\nu_{max}^{KBr}$ ($cm^{-1}$): 1800(sh), 1795(sh), 1780, 1735, 1725(sh)

NMR ($CDCl_3$) δ: 7.82(4H, m), 6.40(1H, d, J = 5Hz), 5.70(1H, d, J = 6Hz), 3.87 - 4.20(2H, m), 3.37(3H, s), 1.1 - 2.2(2H, m), 1.57(9H, s)

4-2) Preparation of (±)-cis-7β-phthalimido-4α-methoxy-1-azabicyclo [4,2,0] oct-2-en-8-oxo-2-carboxylic acid:

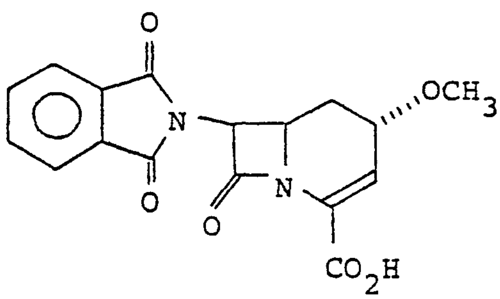

At first, 150 mg of tertiary butyl ester of (±)-cis-7β-phthalimido-4α-hydroxy-1-azabicyclo [4,2,0] oct-2-en-8-oxo-2-carboxylic acid, prepared in 4-1), is dissolved in 2 ml of anhydrous methylene chloride, and then 2 ml of trifluoroacetic acid is added thereto. The mixture is allowed to stand at room temperature for one hour. The reaction mixture is concentrated under reduced pressure. After ethyl acetate is further added thereto, the resulting mixture is twice concentrated under reduced pressure. The residue is washed with ether to obtain 119 mg of the desired compound as white powder (92.0%).

IR $\nu_{max}^{KBr}$ ($cm^{-1}$): 1805(sh), 1800, 1790, 1730(sh), 1725

NMR ($CDCl_3$-$CD_3OD$) δ: 7.85(4H, m), 6.55(1H, d, J = 5Hz), 5.75(1H, d, J = 5.5Hz), 3.9 - 4.3 (2H, m), 3.40(3H, s), 1.2 - 2.3 (2H, m)

4-3) Preparation of (±)-cis-7β-amino-4α-methoxy-1-azabicyclo [4,2,0] oct-2-en-8-oxo-2-carboxylic acid:

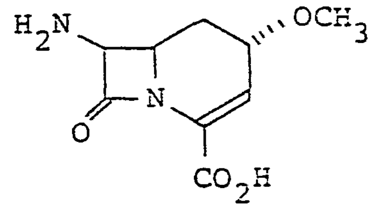

At first, 119 mg of (±)-cis-7β-phthalimido-4α-methoxy-1-azabicyclo [4,2,0] oct-2-en-8-oxo-2-carboxylic acid, prepared in 4-2), is dissolved in 1.9 ml of an aqueous 0.2 N sodium bicarbonate solution, a solution of 30 μl of hydrazine hydrate in 270 μl of water is added thereto with ice cooling over 15 minutes, and the resulting mixture is subjected to reaction as such for one hour. The reaction mixutre is adjusted to pH 1.5 with 1 N hydrochloric acid, and stirred at room temperature for 2 hours. The deposited crystals are filtered off, and the filtrate is adjusted to pH 3.5 with 0.2 N sodium bicarbonate. The resulting solution is concentrated to 2 ml under reduced pressure, and the concentrate is purified twice with Diaion HP-10 (30 ml). The fractions containing the desired compound, as eluted with water, are recovered, and the solvent is distilled off under reduced pressure to obtain 52 mg of white powder (yield: 70.5%).

IR $\nu_{max}^{KBr}$ ($cm^{-1}$): 1800, 1790(sh), 1620(1540 - 1590)

NMR($D_2O$)δ: 6.23(1H, d, J = 5.4Hz), 4.92(1H, d, J = 5.4Hz), 3.9 - 4.3(2H, m), 3.47(3H, s), 2.3 - 2.5(1H, m), 1.71(1H, ddd, J = 13.8, 4.2, 3.9Hz)

4-4) Preparation of (±)-cis-7β-phenylacetamido-4α-methoxy-1-azabicyclo [4,2,0] oct-2-en-8-oxo-2-carboxylic acid:

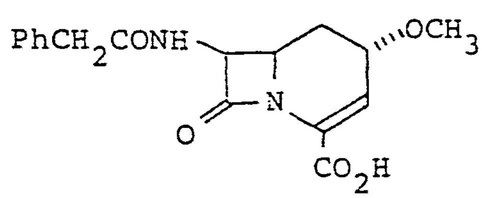

At first, 420 mg of (±)-cis-7β-amino-4α-methoxy-1-azabicyclo [4,2,0] oct-2-en-8-oxo-2-carboxylic acid, prepared in 4-3), is dissolved in 80 ml of an aqueous 50% tetrahydrofuran solution, and the solution is adjusted to pH 7.5 with sodium bicarbonate with ice cooling and stirring. Then, 7 ml of a tetrahydrofuran solution containing 2.3 ml of phenylacetyl chloride is added thereto dropwise at the same temperature, while keeping the reaction solution at pH 7.3 - 7.5 with a saturated aqueous sodium bicarbonate solution. Then, the mixture is stirred at the same temperature for one hour, and the precipitate is filtered off. The filtrate is concentrated under reduced pressure to distill off tetrahydrofuran. The concentrate is washed twice with ethyl acetate, and the aqueous layer is adjusted to pH 1.5 with 1 N hydrochloric acid and then extracted three times with ethyl acetate. After the solvent is distilled off, the residue is dissolved in methanol, adjusted to pH 7.8 with a saturated aqueous sodium bicarbonate solution, and methanol is distilled off under reduced pressure.

The resulting aqueous solution is passed through a column of 200 ml of Diaion HP-10, and the column is then washed with 500 ml of water. Fractions containing the desired compound, as eluted with water-methanol (10 : 1 V/V) are recovered, and the solvent is distilled off under reduced pressure. The residue is dried in vacuo in the pressure of phosphorus pentoxide to obtain 480 mg of the desired compound as while powder (yield: 72.1%).

Physical properties of the compound are as follows:

IR $\nu_{max}^{KBr}$ ($cm^{-1}$): 1770(sh), 1760, 1660, 1600

NMR ($CD_3OD$) $\delta$: 7.30(5H, brs), 6.23(1H, d, J = 5.5Hz), 5.35(1H, d, J = 5.4Hz), 3.7 - 4.0(2H, m), 3.60(2H, s), 3.37(3H, s), 1.80 - 2.17 (1H, m), 1.27 - 1.67(1H, m)

4-5) Preparation of (4S, 6R, 7S)-7-amino-4-methoxy-1-azabicyclo [4,2,0] oct-2-en-8-oxo-2-carboxylic acid:

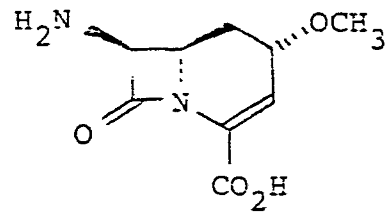

(1) Preparation of cell fracture mixture

(a) Culturing of a microorganism capable of optically selectively removing an acyl group.

Kluyvera citrophila ATCC 21285 [for whose bacteriological disclosure, reference should be made to J. General Applied Microbiology 3 28 - 31 (1957)] is used as a seed microorganism.

An aqueous solution containing 1% polypeptone, 1% yeast extract, 0.5% meat extract, 0.5% sodium glutamate, and 0.25% sodium chloride, adjusted to pH 7.0 with 5 N NaOH is used as a seed medium. One loopful of the seed microorganism is inoculated in 10 ml of the seed medium in a 50ml-large test tube, and cultured at 30°C with shaking for 24 hours. The entire amount of the seed medium is inoculated in 300 ml of a fermentation medium in a 2 ℓ-ribbed Erlenmeyer flask, and cultured at 30°C with shaking.

The fermentation medium has the same composition as that of the seed medium.

(b) Preparation of cell fracture mixture

After the fermentation culturing for 24 hours, cells are separated from the fermentation liquor by centrifugation, and washed twice with 50 ml of an aqueous 0.9% sodium chloride solution. The cells are suspended in a 1/30 M phosphate buffer. The cell concentration is 40 mg/ml, where the weight of cells is in terms of that of dried cells. Then, 10 ml of the suspension is put in a

50 ml-large test tube, and then fractured with ultrasonic wave treatment at 200 W for 2 minutes to obtain a cell fracture mixture. For the treatment, an ultrasonic wave generator, model UR200P, made by Tomy Seiko Co., is used.

(2) Preparation of substrate solution

At first, 480 mg of sodium salt of (±)-cis-7β-phenylacetamido-4α-methoxy-1-azabicyclo [4,2,0] oct-2-en-8-oxo-2-carboxylic acid is added to 22 ml of a 1/30 M phosphate buffer (pH 6.5). At that time, the compound is not dissolved. Then, 2N-NaOH is added thereto by portions and pH is adjusted again to 6.5, whereby the compound is dissolved. Finally, deionized water is added thereto to make the volume 24 ml.

(3) Enzymatic reaction

At first, 24 ml of the cell fracture mixture is added to 24 ml of the substrate solution, and the mixture is subjected to enzymatic reaction at 30°C for 80 minutes.

(4) Isolation and purification of desired compound

After the reaction, cells are removed from the reaction mixture by centrifugation, and the supernatant is adjusted to pH 3.0 with 2 N hydrochloric acid. Then, the supernatant is charged onto a column of 200 ml of Diaion HP-10 (column diameter 2.6 cm, height 38 cm), the column is eluted with deionized water, and the eluate is separated in 5 ml portions. The desired compound elutes in fractions obtained between 190 ml and 230 ml. The fractions are concentrated under reduced pressure and freeze dried to obtain 107.7 mg of white powder (71.1%).

Physical properties of the compound are as follows.

$$[\alpha]_D^{20^\circ} = -86.0^\circ \quad (c = 0.25,\ 1\ M\ \text{phosphate buffer, pH 7})$$

IR and NMR spectra are quite identical with those of the corresponding dl isomer.

Reference Example 5

Preparation of tertiary butyl ester of (±)-cis-7β-phthalimido-1-azabicyclo [4,2,0] oct-2-en-8-oxo-2-carboxylic acid:

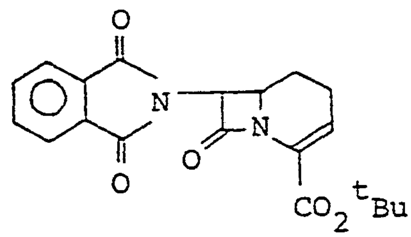

5-1) Preparation of tertiary butyl ester of cis-4-(3,3-dimethoxy-1-propen-1-yl)-3-phthalimido-2-oxoazetidin-1-yl-α-diethylphosphonoacetic acid:

At first, 20 g (74.8 mM) of tertiary butyl ester of α-amino-α-diethylphosphonoacetic acid is dissolved in 160 ml of ethyl acetate, and 11.43 g (82.3 mM) of 4,4-dimethyl-trans-2-butenal is added to the solution. The solution is heated at 50°C for 10 minutes. The solvent is concentrated under reduced pressure at a bath temperature of 50°C, whereby a Schiff base is obtained as an oily matter. Then, 250 ml of anhydrous methylene chloride is added to the oily matter, and then 12.7 ml (89.8 mM) of triethylamine is added to the resulting solution with ice cooling and stirring. Then, 18.4 g (82.3 mM) of phthalylglycine acid chloride dissolved in 60 ml of methylene chloride is added dropwise to the reaction mixture over one hour. After the dropwise addition, ice water bath is removed, and the mixture is continuously stirred as such up to room temperature for 2 hours. Then, the insoluble matters in the reaction mixture is filtered off, and the filtrate is washed with 100 ml of a saturated aqueous sodium chloride solution, twice with a mixed solution of 25 ml of a saturated aqueous sodium hydrogen sulfite solution and 25 ml of water, and then twice with a saturated aqueous sodium chloride solution. The organic layer is dried over anhydrous sodium sulfate, and the solvent is distilled off to obtain 46 g of oily

matter (hereinafter referred to as crude acetal compound). The crude acetal compound is purified by silica gel chromatography (Wako gel C-200, 2 ℓ; elution n-hexane : ethyl acetate = 1 : 2 V/V) to obtain 31.8 g of the desired compound (as a diastereomer mixture of approximately 1 : 1) as crystalline oily matter (yield : 75%).

NMR $\delta(CDCl_3)$(ppm): 1.30 - 1.53(m, 15H), 2.98(s, 3/2H), 3.01(s, 3/2H), 3.08(s, 3/2H), 3.10(s, 3/2H), 4.06 - 4.41(m, 4H), 4.61(d, J = 5Hz, 1/2H), 4.62(d, J = 5Hz, 1/2H), 4.81 - 5.04(m, 1H), 4.99(d, J = 24Hz, 1/2H), 5.02(d, J = 24Hz, 1/2H), 5.51 - 5.74(m, 2H), 5.9 - 6.2(m, 1H), 7.68 - 7.92(m, 4H)

IR $\nu_{max}^{CHCl_3}$ ($cm^{-1}$): 1790(sh), 1780, 1775, 1730

5-2) Preparation of tertiary butyl ester of cis-4-(3-oxo-1-propen-1-yl)-3-phthalimido-2-oxoazetidin-1-yl-α-diethylphosphonoacetic acid:

At first, 7.1 g of p-toluenesulfonic acid monohydrate is added to a solution of 46 g of the crude acetal compound obtained in 5-1) in a mixed solvent of 400 ml of methylene chloride and 20 ml of acetone with ice cooling and stirring, and the mixture is continuously stirred as such for one hour and 30 minutes. Then, the water is added to the reaction mixture, and the organic layer is washed with water, twice with a saturated aqueous sodium chloride solution, once with a saturated aqueous sodium bicarbonate solution, and then three times with a saturated aqueous sodium chloride solution. The organic layer is dried over anhydrous sodium sulfate, and then the solvent is distilled off to obtain 35 g of oily matter (hereinafter referred to as crude aldehyde compound). The crude aldehyde compound is purified by silica gel chromatography (Wako gel C-200, 600 ml; elution n-hexane : ethyl acetate = 1 : 1 V/V), whereby 28.3 g of the desired compound as a diastereomer mixture is obtained as crystalline oily matter.

Physical properties of the compound are as follows:

IR $\nu_{max}^{CHCl_3}$ ($cm^{-1}$): 1790(sh), 1780, 1730, 1695

NMR ($CDCl_3$)$\delta$: 9.5(1H, d, J = 8.0Hz), 7.8(4H, br), 6.9 - 7.4(1H, m), 5.9 - 6.3(1H, m), 5.7(1H, m), 5.1(1H, d, J = 23Hz), 4.0 - 4.5(5H, m), 1.5(9H, s), 1.4(6H, t, J = 7Hz).

5-3) Preparation of tertiary butyl ester of (±)-cis-7β-phthalimido-1-azabicyclo [4,2,0] oct-2-en-8-oxo-2-carboxylic acid:

At first, 60 g of tertiary butyl ester of cis-4-(3-oxo-1-propen-1-yl)-3-phthalimido-2-oxoazetidin-1-yl-α-diethylphosphonoacetic acid, prepared in 5-2), is dissolved in 600 ml of dimethoxyethane, and 30 g of 5% palladium carbon is added thereto. A hydrogen gas is introduced in the mixture at 40°C with stirring for 3 hours. After the reaction, the catalyst is filtered off, and then 20 g of diazabicyclooctane is added to the reaction mixture, which is then allowed to stand at room temperature overnight. Then, 1 ℓ of chloroform is added to the reaction mixture, which is then washed with diluted hydrochloric acid and with water, and concentrated under reduced pressure. The residue is treated with ethyl acetate, whereby crystals are obtained in a colorless, needle state. By filtration, 29.6 g of the desired compound is obtained (70%).

Physical properties of the compound are as follows:

IR $\nu_{max}^{CHCl_3}$ ($cm^{-1}$): 1800, 1780, 1735, 1635

NMR ($CDCl_3$)$\delta$: 7.82(4H, m), 6.35(1H, m), 5.64(1H, d, J = 5.0Hz), 3.88(1H, m), 1.67 - 2.58(4H, m), 1.57(9H, s)

What is Claimed is:

1. A β-lactam compound represented by the general formula (I):

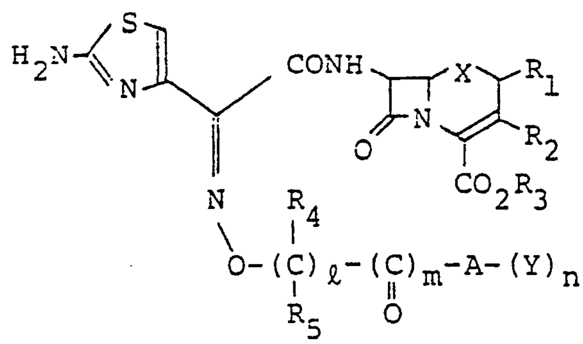

(I)

{wherein X represents $CH_2$, S or O; Y represents a hydroxyl group, lower alkanoyloxy group; A represents a phenyl group, naphthyl group, respectively substituted by $(Y)_n$; $R_1$ represents a hydrogen atom, hydroxyl group, methoxy group or lower alkyl group; $R_2$ represents a hydrogen atom, halogen atom, methoxy group or $CH_2R'_2$ [wherein $R'_2$ represents a hydrogen atom, azido group, lower alkanoyloxy group, carbamoyloxy group, pyridinium group, substituted pyridinium group, or substituted or unsubstituted heterocyclic thio group (wherein the term "heterocyclic" means a 5-membered or 6-membered heterocyclic group having 1 to 4 hetero atoms of O, S and N)]; $R_3$ represents a hydrogen atom, alkali metal, alkaline earth metal, organic ammonium group or ester residue; $R_4$ and $R_5$ each represent a hydrogen atom, lower alkyl group or cycloalkylidene group together with the carbon atom combined therewith; $\ell$ represents an integer of 0, 1, 2 or 3; m represents an integer of 0 or 1; and n represents an integer of 1, 2, 3, 4 or 5}.

2. A β-lactam compound according to claim 1 wherein $\ell$ is 0 and m is 1, that is, represented by the general formula (VIII):

BAD ORIGINAL

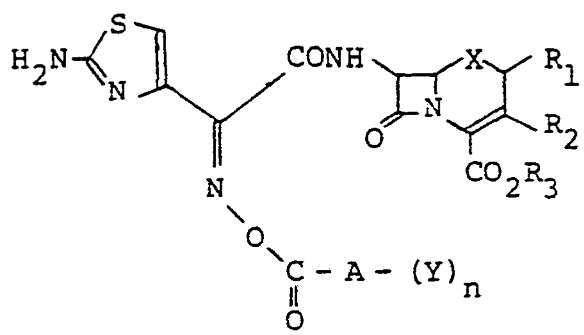

( VIII )

(wherein X, Y, A, $R_1$, $R_2$, $R_3$ and n have the same meanings as defined above).

3. A β-lactam compound according to claim 2 wherein X is $CH_2$, A is a phenyl group substituted by $(Y)_n$, Y is a hydroxyl group or lower alkanoyloxy group, $R_1$ is a hydrogen atom, hydroxyl group or methoxy group, and $R_2$ is a hydrogen atom.

4. A β-lactam compound according to claim 2 wherein X is S, A is a phenyl group substituted by $(Y)_n$, Y is a hydroxyl group or lower alkanoyloxy group, $R_1$ is a hydrogen atom, and $R_2$ is $CH_2R_2'$ (where $R_2'$ is a lower alkanoyloxy group, pyridinium group, substituted pyridinium group, or substituted or unsubstituted heterocyclic thio group).

5. A β-lactam compound according to claim 4 wherein said substituted or unsubstituted heterocyclic thio group is a tetrazolylthio group, or a tetrazolylthio group substituted by a lower alkyl group having 1 to 5 carbon atoms or $-(CH_2)_qCO_2H$ (wherein q is an integer of 1 to 3).

6. A β-lactam compound according to claim 1 wherein ℓ is 1 and m is 1, that is, represented by the general formula (IX):

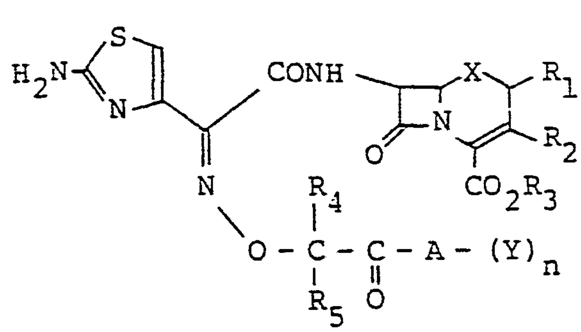

( IX )

(wherein X, Y, A, $R_1$, $R_2$, $R_3$, $R_4$, $R_5$ and n have the same meanings as defined above).

7. A β-lactam compound according to claim 6 wherein X is $CH_2$, $R_4$ and $R_5$ each represent a hydrogen atom or lower alkyl group, A is a phenyl group substituted by $(Y)_n$, Y is a hydroxyl group or lower alkanoyloxy group, $R_1$ is a hydrogen atom, hydroxyl group or methoxy group, and $R_2$ is a hydrogen atom.

8. A β-lactam compound according to claim 6 wherein X is S, $R_4$ and $R_5$ each represent a hydrogen atom or lower alkyl group, A is a phenyl group substituted by $(Y)_n$, Y is a hydroxyl group or lower alkanoyloxy group, $R_1$ is a hydrogen atom, and $R_2$ is $CH_2R_2'$ (wherein $R_2'$ is a lower alkanoyloxy group, pyridinium group, substituted pyridinium group, or substituted or unsubstituted heterocyclic thio group.

9. A β-lactam compound according to claim 1 wherein ℓ is 1 and m is 0, that is, represented by the general formula (X):

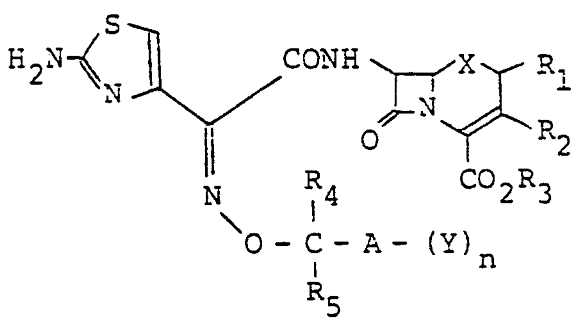

(X)

(wherein X, Y, A, $R_1$, $R_2$, $R_3$, $R_4$, $R_5$ and n have the same meanings as defined above).

10. A β-lactam compound according to claim 9 wherein X is $CH_2$, $R_4$ and $R_5$ are a hydrogen atom, A is a phenyl group substituted by $(Y)_n$, Y is a hydroxyl group or lower alkanoyloxy group, $R_1$ is a hydrogen atom, hydroxyl group or methoxy group, and $R_2$ is a hydrogen atom.

11. A carboxylic acid represented by the general formula (II):

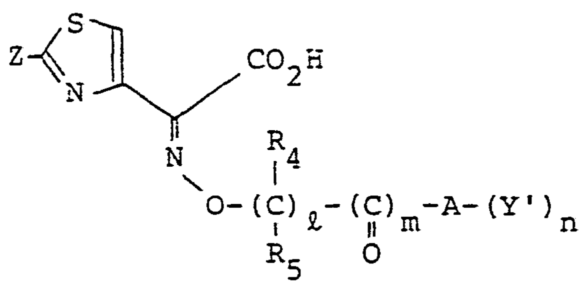

( II )

(wherein Z represents an amino group or protected amino group, Y' represents the same Y as defined above, a protected hydroxyl group or protected amino group, and A, $R_4$, $R_5$, ℓ, m and n have the same meanings as defined above), and its reactive derivative.

12. A compound according to claim 11 wherein ℓ is 0 and m is 1, that is, a carboxylic acid represented by the general formula (XI):

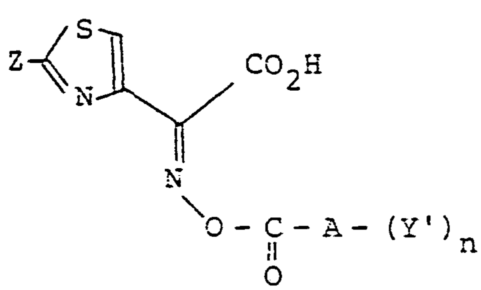

( XI )

(wherein Z, A, Y' and n have the same meanings as defined above) or its reactive derivative.

BAD ORIGINAL

13. A compound according to claim 12 wherein A is a phenyl group substituted by $(Y')_n$ and Y' is a hydroxyl group, lower alkanoyloxy group or protected hydroxyl group.

14. A compound according to claim 11 wherein ℓ is 1 and m is 1, that is, a carboxylic acid represented by the general formula (XII):

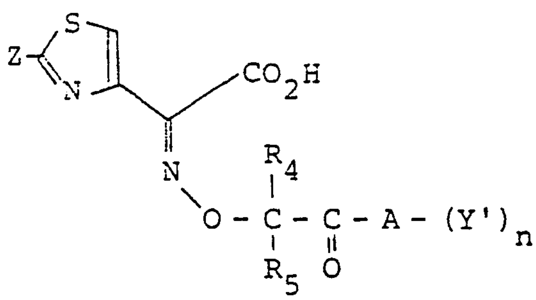

(XII)

(wherein Z, $R_4$, $R_5$, A, Y' and n have the same meanings as defined above) or its reactive derivative.

15. A compound according to claim 14 wherein $R_4$ and $R_5$ each represent a hydrogen atom or lower alkyl group, A is a phenyl group substituted by $(Y')_n$, and Y' is a hydroxyl group, lower alkanoyloxy group or protected hydroxyl group.

16. A compound according to claim 11 wherein ℓ is 1 and m is 0, that is, a carboxylic acid represented by the general formula (XIII):

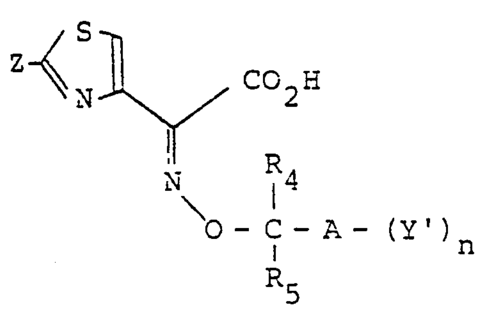

(XIII)

(wherein Z, $R_4$, $R_5$, A, Y' and n have the same meanings as defined above).

BAD ORIGINAL

17. A compound according to claim 16 wherein $R_4$ and $R_5$ are a hydrogen atom, A is a phenyl group substituted by $(Y')_n$, Y' is a hydroxyl group, lower alkanoyloxy group or protected hydroxyl group.

18. Pharmaceutical compositions with antimicrobial activity comprising a compound according to any of claims 1 to 10 together with conventional diluent(s), and/or carrier(s) and/or adjuvant(s).

18. An antimicrobial pharmaceutical composition which comprises an effective antimicrobial amount of a compound according to any of claims 1 to 10 and at least one member of the group consisting of pharmaceutically acceptable diluents, carriers and adjuvants.

BAD ORIGINAL

European Patent Office

# EUROPEAN SEARCH REPORT

0075805

Application number

EP 82 10 8606.3

| Category | DOCUMENTS CONSIDERED TO BE RELEVANT<br>Citation of document with indication, where appropriate, of relevant passages | Relevant to claim |
|---|---|---|
| A | GB - A - 2 017 702 (ROUSSEL-UCLAF)<br>* claim 1 *<br>-- | 1 |
| A | DE - A1 - 2 716 677 (HOECHST AG)<br>* claim 1 *<br>-- | 1 |
| D,A | EP - A1 - 0 014 476 (KYOWA HAKKO KOGYO CO. LTD)<br>* claim 1 *<br>-- | 1 |
| P,A | GB - A - 2 083 820 (FUJISAWA PHARMA-CUETICAL CO. LTD.)<br>* claim 1 *<br>-- | 11 |
| D,A | US - A - 4 165 430 (GLAXO LABORATORIES)<br>---- | |

CLASSIFICATION OF THE APPLICATION (Int. Cl. 3)

C 07 D 501/20
C 07 D 471/04
C 07 D 501/34
C 07 D 501/36
C 07 D 501/56
C 07 D 277/40
C 07 D 277/42
C 07 D 277/44
A 61 K 31/425
A 61 K 31/435
A 61 K 31/545
//(C 07D 471/04, 221/00, 205/00)

TECHNICAL FIELDS SEARCHED (Int.Cl. 3)

C 07 D 277/00
C 07 D 471/04
C 07 D 501/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 14-12-1982 | PHILLIPS |